(19) Europäisches Patentamt
European Patent Office
Office européen des brevets

(11) EP 2 179 992 A1

# (12) EUROPÄISCHE PATENTANMELDUNG

(43) Veröffentlichungstag:
**28.04.2010 Patentblatt 2010/17**

(51) Int Cl.:
***C07D 239/47*** *(2006.01)* ***A61K 31/505*** *(2006.01)*
***A61P 35/00*** *(2006.01)*

(21) Anmeldenummer: **08167115.8**

(22) Anmeldetag: **21.10.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(71) Anmelder: **Bayer Schering Pharma Aktiengesellschaft**
**13353 Berlin (DE)**

(72) Erfinder:
- **Lücking, Ulrich**
**10317 Berlin (DE)**
- **Siemeister, Gergard**
**13503 Berlin (DE)**
- **Lienau, Philip**
**10997 Berlin (DE)**
- **Jautelat, Rolf**
**16548 Glienicke/Nordbahn (DE)**
- **Schulze, Julia**
**10407 Berlin (DE)**

(54) **Sulfonsubstituierte Anlinopyrimidinderivative als CDK-Inhibitoren, deren Herstellung und Verwendung als Arzneimittel**

(57) Die Erfindung betrifft sulfonsubsituierte Anilino-Pyrimidinderivate der Formel (I),.

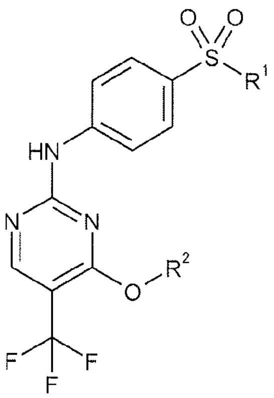

(I),

deren Verfahren zur Herstellung, sowie deren Verwendung als Medikament zur Behandlung verschiedener Erkrankungen.

EP 2 179 992 A1

**Beschreibung**

Die vorliegende Erfindung betrifft sulfonsubsituierte Anilino-Pyrimidinderivate, deren Verfahren zur Herstellung, sowie deren Verwendung als Medikament zur Behandlung verschiedener Erkrankungen.

Die Zyklin-abhängigen Kinasen (cyclin-dependent kinase, CDK) sind eine Enzymfamilie, die eine wichtige Rolle bei der Regulation des Zellzyklusses spielt und somit ein besonders interessantes Ziel für die Entwicklung kleiner inhibitorischer Moleküle darstellt. Selektive Inhibitoren der CDKs können zur Behandlung von Krebs oder anderen Erkrankungen, die Störungen der Zellproliferation zur Ursache haben, verwendet werden.

Pyrimidine und Analoga sind bereits als Wirkstoffe beschrieben wie beispielsweise die 2-Anilino-Pyrimidine als Fungizide (DE 4029650) oder substituierte Pyrimidinderivate zur Behandlung von neurologischen oder neurodegenerativen Erkrankungen (WO 99/19305). Als CDK-Inhibitoren werden unterschiedlichste Pyrimidinderivate beschrieben, beispielsweise 2-Amino-4-substituierte Pyrimidine (WO 01/ 14375), Purine (WO 99/02162), 5-Cyano-Pyrimidine (WO 02/04429), Anilinopyrimidine (WO 00/12486) und 2-Hydroxy-3-N,N-dimethylaminopropoxy-Pyrimidine (WO 00/39101).

Insbesondere wurden in WO 02/096888 und WO 03/076437 Pyrimidinderivate offenbart, die inhibitorische Wirkungen bezüglich CDKs aufweisen.

WO 2005/037800 offenbart offene sulfoximinsubsituierte Anilino-Pyrimidinderivate als Inhibitoren der Zyklin-abhängigen Kinasen. Beispielhaft belegt sind Strukturen, die in der 5-Position des Pyrimidins entweder nicht oder mit Halogen, insbesondere mit Brom substituiert sind. Einen 5-Trifluormethylsubstituenten weist keine der spezifisch offenbarten Strukturen auf.

WO 2003/032997 offenbart sulfonsubstituierte Anilinopyrimidine, für die allerdings in der Position 4 des Pyrimidins obligat ein stickstoffhaltiger Rest vorgesehen ist.

Die den erfindungsgemäßen Strukturen am nächsten kommende spezifisch offenbarte Struktur ist die Struktur 692 des Beispiels 1.

Ausgehend von diesem Stand der Technik besteht die Aufgabe der vorliegenden Erfindung Verbindungen bereitzustellen, die die Aktivität der Zyklin-abhängigen Kinasen stärker inhibieren als die Verbindungen des Standes der Technik. Weiterhin sollen die Verbindungen selektiver gegenüber der Inhibition der VEGF-Rezeptorkinase-2 (VEGF-R2) sein. Die Verbindungen sollen gut permeabel in absorptiver Richtung und gering permeabel in Efflux-Richtung sein. Insbesondere sollen die Verbindungen auch in Chemotherapie-resistenten Tumorzellen stark antiproliferativ wirken.

Es wurde nun gefunden, dass Verbindungen der allgemeinen Formel (I)

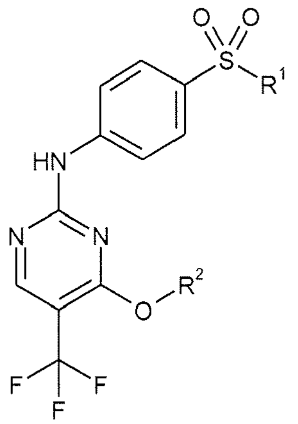

(I),

in der

| | |
|---|---|
| $R^1$ | für einen $C_1$-$C_6$-Alkyl-, $C_2$-$C_6$-Alkenyl-, $C_2$-$C_6$-Alkinylrest oder einen $C_3$-$C_7$-Cycloalkyl oder Phenylring steht, jeweils gegebenenfalls mit Hydroxy, -$NR^3R^4$, Cyano, Halogen, -$CF_3$, $C_1$-$C_6$-Alkoxy, -$OCF_3$ und/oder $C_1$-$C_6$-Alkyl ein- oder mehrfach, gleich oder verschieden substituiert, und |
| $R^2$ | für einen $C_1$-$C_{10}$-Alkyl-, $C_3$-$C_{10}$-Alkenyl- oder $C_3$-$C_{10}$-Alkinylrest oder einen $C_3$-$C_7$-Cycloalkylring steht, jeweils gegebenenfalls ein- oder mehrfach, gleich oder verschieden substituiert mit a) Halogen, Hydroxy, -$NR^3R^4$, Cyano, -$CF_3$, -$OCF_3$, und/oder b) $C_1$-$C_6$-Alkoxy, $C_1$-$C_6$-Alkyl, $C_2$-$C_6$-Alkenyl, $C_2$-$C_6$-Alkinyl, oder $C_3$- $C_8$-Cycloalkyl, -O-$CH_2$-Phenyl, $C_n$-Alkoxycarbonyl, jeweils gegebenenfalls selbst ein- oder mehrfach, gleich oder verschieden mit Halogen, Hydroxy, einem $C_1$-$C_6$-Alkyl, $C_1$-$C_6$- Alkoxy, -$NR^3R^4$, -$CF_3$ und/oder -$OCF_3$ substituiert, und |
| $R^3$ und $R^4$ | unabhängig voneinander für Wasserstoff und/oder einen $C_1$-$C_6$- Alkylrest, $C_2$-$C_6$-Alkenylrest, $C_3$-$C_8$-Cy- |

cloalkyl- und/oder Phenylring, einen Heterocyclylring mit 3 bis 8 Ringatomen und/oder einen monocyclischen Heteroarylring, gegebenenfalls mit Hydroxy, $-NR^5R^6$, Cyano, Halogen, $-CF_3$, $C_1$-$C_6$-Alkoxy und/oder $-OCF_3$ ein- oder mehrfach, gleich oder verschieden substituiert sind, oder

$R^3$ und $R^4$ gemeinsam mit dem Stickstoffatom einen 5- bis 7-gliedrigen Ring bilden, der gegebenenfalls zusätzlich zum Stickstoffatom 1 oder 2 weitere Heteroatome enthält und der mit Hydroxy, $-NR^5R^6$, Cyano, Halogen, $-CF_3$, $C_1$-$C_6$-Alkoxy und/oder $-OCF_3$ ein- oder mehrfach, gleich oder verschieden substituiert sein kann, und

$R^5$ und $R^6$ unabhängig voneinander für Wasserstoff oder einen $C_1$-$C_6$-Alkylrest stehen, der gegebenenfalls mit Hydroxy, Cyano, Halogen, $-CF_3$, $C_1$-$C_6$-Alkoxy und/oder $-OCF_3$ ein- oder mehrfach, gleich oder verschieden substituiert ist.

sowie deren Salze, Diatereomere und Enantiomere.

Der Erfindung liegen folgende Definitionen zu Grunde: $C_n$-Alkyl:

Monovalenter, geradkettiger oder verzweigter, gesättigter Kohlenwasserstoffrest mit n Kohlenstoffatomen.

Ein $C_1$-$C_6$ Alkylrest umfasst unter anderem beispielsweise:

Methyl-, Ethyl-, Propyl-, Butyl-, Pentyl-,Hexyl-, *iso*-Propyl-, *iso*-Butyl-, *sec*-Butyl, *tert*-Butyl-, *iso*-Pentyl-, 2-Methylbutyl-, 1-Methylbutyl-, 1-Ethylpropyl-, 1,2-Dimethylpropyl, *neo*-Pentyl-, 1,1-Dimethylpropyl-, 4-Methylpentyl-, 3-Methylpentyl-, 2-Methylpentyl-, 1-Methylpentyl-, 2-Ethylbutyl-, 1-Ethylbutyl-, 3,3-Dimethylbutyl-, 2,2-Dimethylbutyl-, 1,1-Dimethylbutyl-, 2,3-Dimethylbutyl-, 1,3-Dimethylbutyl- 1,2-Dimethylbutyl-.

Bevorzugt ist ein Methyl-, Ethyl-, Propyl- oder Isopropylrest.

$C_n$-Alkenyl:

monovalenter, geradkettiger oder verzweigter Kohlenwasserstoffrest mit n Kohlenstoffatomen und mindestens einer Doppelbindung.

Ein $C_2$-$C_{10}$ Alkenylrest umfasst unter anderem beispielsweise:

Vinyl-, Allyl-, (E)-2-Methylvinyl-, (Z)-2-Methylvinyl-, Homoallyl-, (E)-But-2-enyl-, (Z)-But-2-enyl-, (E)-But-1-enyl-, (Z)-But-1-enyl-, Pent-4-enyl-, (E)-Pent-3-enyl-, (Z)-Pent-3-enyl-, (E)-Pent-2-enyl-, (Z)-Pent-2-enyl-, (E)-Pent-1-enyl-, (Z)-Pent-1-enyl, Hex-5-enyl-, (E)-Hex-4-enyl-, (Z)-Hex-4-enyl-, (E)-Hex-3-enyl-, (Z)-Hex-3-enyl-, (E)-Hex-2-enyl-, (Z)-Hex-2-enyl-, (E)-Hex-1-enyl-, (Z)-Hex-1-enyl-, Isopropenyl-, 2-Methylprop-2-enyl-, 1-Methylprop-2-enyl-, 2-Methylprop-1-enyl-, (E)-1-Methylprop-1-enyl-, (Z)-1-Methylprop-1-enyl-, 3-Methylbut-3-enyl-, 2-Methylbut-3-enyl-, 1-Methylbut-3-enyl-, 3-Methylbut-2-enyl-, (E)-2-Methylbut-2-enyl-, (Z)-2-Methylbut-2-enyl-, (E)-1-Methylbut-2-enyl-, (Z)-1-Methylbut-2-enyl-, (E)-3-Methylbut-1-enyl-, (Z)-3-Methylbut-1-enyl-, (E)-2-Methylbut-1-enyl-, (Z)-2-Methylbut-1-enyl-, (E)-1-Methylbut-1-enyl-, (Z)-1-Methylbut-1-enyl-, 1,1-Dimethylprop-2-enyl-, 1-Ethylprop-1-enyl-, 1-Propylvinyl-, 1-Isopropylvinyl-, 4-Methylpent-4-enyl-, 3-Methylpent-4-enyl-, 2-Methylpent-4-enyl-, 1-Methylpent-4-enyl-, 4-Methylpent-3-enyl-, (E)-3-Methylpent-3-enyl-, (Z)-3-Methylpent-3-enyl-, (E)-2-Methylpent-3-enyl-, (Z)-2-Methylpent-3-enyl-, (E)-1-Methylpent-3-enyl-, (Z)-1-Methylpent-3-enyl-, (E)-4-Methylpent-2-enyl-, (Z)-4-Methylpent-2-enyl-, (E)-3-Methylpent-2-enyl-, (Z)-3-Methylpent-2-enyl-, (E)-2-Methylpent-2-enyl-, (Z)-2-Methylpent-2-enyl-, (E)-1-Methylpent-2-enyl-, (Z)-1-Methylpent-2-enyl-, (E)-4-Methylpent-1-enyl-, (Z)-4-Methylpent-1-enyl-, (E)-3-Methylpent-1-enyl-, (Z)-3-Methylpent-1-enyl-, (E)-2-Methylpent-1-enyl-, (Z)-2-Methylpent-1-enyl-, (E)-1-Methylpent-1-enyl-, (Z)-1-Methylpent-1-enyl-, 3-Ethylbut-3-enyl-, 2-Ethylbut-3-enyl-, 1-Ethylbut-3-enyl-, (E)-3-Ethylbut-2-enyl-, (Z)-3-Ethylbut-2-enyl-, (E)-2-Ethylbut-2-enyl-, (Z)-2-Ethylbut-2-enyl-, (E)-1-Ethylbut-2-enyl-, (Z)-1-Ethylbut-2-enyl-, (E)-3-Ethylbut-1-enyl-, (Z)-3-Ethylbut-1-enyl-, 2-Ethylbut-1-enyl-, (E)-1-Ethylbut-1-enyl-, (Z)-1-Ethylbut-1-enyl-, 2-Propylprop-2-enyl-, 1-Propylprop-2-enyl-, 2-Isopropylprop-2-enyl-, 1-Isopropylprop-2-enyl-, (E)-2-Propylprop-1-enyl-, (Z)-2-Propylprop-1-enyl-, (E)-1-Propylprop-1-enyl-, (Z)-1-Propylprop-1-enyl-, (E)-2-Isopropylprop-1-enyl-, (Z)-2-Isopropylprop-1-enyl-, (E)-1-Isopropylprop-1-enyl-, (Z)-1-Isopropylprop-1-enyl-, (E)-3,3-Dimethylprop-1-enyl-, (Z)-3,3-Dimethylprop-1-enyl-, 1-(1,1-Dimethylethyl)ethenyl.

Bevorzugt ist ein Vinyl- oder Allylrest.

$C_n$-Alkinyl:

Monovalenter, geradkettiger oder verzweigter Kohlenwasserstoffrest mit n Kohlenstoffatomen und mindestens einer Dreifachbindung.

Ein $C_2$-$C_{10}$ Alkinylrest umfasst unter anderem beispielsweise:

Ethinyl-, Prop-1-inyl-, Prop-2-inyl-, But-1-inyl-, But-2-inyl-, But-3-inyl-, Pent-1-inyl-, Pent-2-inyl-, Pent-3-inyl-, Pent-4-inyl-, Hex-1-inyl-, Hex-2-inyl-,
Hex-3-inyl-, Hex-4-inyl-, Hex-5-inyl-, 1-Methylprop-2-inyl-, 2-Methylbut-3-inyl-, 1-Methylbut-3-inyl-, 1-Methylbut-2-inyl-, 3-Methylbut-1-inyl-, 1-Ethylprop-2-inyl-, 3-Methylpent-4-inyl, 2-Methylpent-4-inyl, 1-Methylpent-4-inyl, 2-Methylpent-3-inyl, 1-Methylpent-3-inyl, 4-Methylpent-2-inyl, 1-Methylpent-2-inyl,
4-Methylpent-1-inyl, 3-Methylpent-1-inyl, 2-Ethylbut-3-inyl-, 1-Ethylbut-3-inyl-, 1-Ethylbut-2-inyl-, 1-Propylprop-2-inyl-, 1-Isopropylprop-2-inyl-, 2,2-Dimethylbut-3-inyl-, 1,1-Dimethylbut-3-inyl-, 1,1-Dimethylbut-2-inyl- oder 3,3-Dimethylbut-1-inyl-.

Bevorzugt ist ein Ethinyl-, Prop-1-inyl- oder Prop-2-inyl-rest.

$C_n$-Cycloalkyl:

Monovalenter, cyclischer Kohlenwasserstoffring mit n Kohlenstoffatomen. $C_3$-$C_7$-Cyclolalkylring umfasst:

Cyclopropyl, Cyclobutyl, Cyclopentyl, Cyclohexyl- und Cycloheptyl.

Bevorzugt ist ein Cyclopropyl-, Cylopentyl- oder ein Cyclohexylring.

$C_n$-Alkoxy:

Geradkettiger oder verzweigter $C_n$-Alkyletherrest der Formel -OR mit R= $C_n$-Alkyl.

$C_n$-Alkoxycarbonyl

$C_n$-Alkoxycarbonyl steht für die Gruppe -C(O)-O-$C_n$-Alkyl.
In der Regel ist p gleich 1 bis 6, bevorzugt 1 bis 4, und besonders bevorzugt 1 bis 3. Beispielhaft und vorzugsweise seien genannt:

Methoxycarbonyl, Ethoxycarbonyl, Propoxycarbonyl, Isopropoxycarbonyl, tert.-Butoxycarbonyl, n-Pentoxycarbonyl und n-Hexoxycarbonyl.

Halogen

Die Bezeichnung Halogen umfasst Fluor, Chlor, Brom und Jod.
Bevorzugt ist Fluor.
In der allgemeinen Formel (I) kann $R^1$ stehen für:

einen $C_1$-$C_6$-Alkyl-, $C_2$-$C_6$-Alkenyl-, $C_2$-$C_6$-Alkinylrest oder einen $C_3$-$C_7$-Cycloalkyl oder Phenylring, jeweils gegebenenfalls mit Hydroxy, -$NR^3R^4$, Cyano, Halogen, -$CF_3$, $C_1$-$C_6$-Alkoxy, -$OCF_3$ und/oder $C_1$-$C_6$-Alkyl ein- oder mehrfach, gleich oder verschieden substituiert.

Bevorzugt steht $R^1$ für einen $C_1$-$C_6$-Alkyl-, $C_2$-$C_6$-Alkenyl-, $C_2$-$C_6$-Alkinylrest oder einen $C_3$-$C_7$-Cycloalkyl oder Phenylring, jeweils gegebenenfalls mit Hydroxy, Cyano, Halogen, -$CF_3$, $C_1$-$C_6$-Alkoxy, -$OCF_3$ und/oder $C_1$-$C_6$-Alkyl ein- oder mehrfach, gleich oder verschieden substituiert.
Mehr bevorzugt steht $R^1$ für einen $C_1$-$C_6$-Alkyl- oder $C_2$-$C_6$-Alkenylrest oder einen $C_3$-$C_7$-Cycloalkyl- oder Phenylring, jeweils gegebenenfalls mit Hydroxy, Cyano, Halogen und/oder $C_1$-$C_6$-Alkyl ein- oder mehrfach, gleich oder verschieden substituiert.
Mehr bevorzugt steht $R^1$ für einen $C_1$-$C_4$-Alkyl- oder $C_2$-$C_4$-Alkenylrest oder einen $C_3$-$C_5$-Cycloalkyl- oder Phenylring, jeweils gegebenenfalls mit Hydroxy, Cyano, Halogen und/oder $C_1$-$C_3$-Alkyl ein- oder mehrfach, gleich oder verschieden substituiert.
Außerordentlich bevorzugt steht $R^1$ für eine Methylgruppe oder für einen Cyclopropylring.
In der allgemeinen Formel (I) kann $R^2$ stehen für:

einen $C_1$-$C_{10}$-Alkyl-, $C_3$-$C_{10}$-Alkenyl- oder $C_3$-$C_{10}$-Alkinylrest oder einen $C_3$-$C_7$-Cycloalkylring, jeweils gegebenenfalls ein- oder mehrfach, gleich oder verschieden substituiert mit

a) Halogen, Hydroxy, -$NR^3R^4$, Cyano, -$CF_3$, -$OCF_3$, und/oder
b) $C_1$-$C_6$-Alkoxy, $C_1$-$C_6$-Alkyl, $C_2$-$C_6$-Alkenyl, $C_2$-$C_6$-Alkinyl, oder $C_3$-$C_8$-Cycloalkyl, -O-$CH_2$-Phenyl, $C_n$-Alkoxycar-

bonyl, jeweils gegebenenfalls selbst ein- oder mehrfach, gleich oder verschieden mit Halogen, Hydroxy, einem $C_1$-$C_6$-Alkyl, $C_1$-$C_6$-Alkoxy, $-NR^3R^4$, $-CF_3$ und/oder $-OCF_3$ substituiert.

Bevorzugt steht $R^2$ für einen $C_1$-$C_{10}$-Alkyl-, $C_3$-$C_{10}$-Alkenyl- oder $C_3$-$C_{10}$-Alkinylrest oder einen $C_3$-$C_7$-Cycloalkylring, jeweils gegebenenfalls ein- oder mehrfach, gleich oder verschieden substituiert mit Halogen, Hydroxy, Cyano, $-CF_3$, $-OCF_3$, und/oder $C_1$-$C_6$-Alkoxy, $C_1$-$C_6$-Alkyl, jeweils gegebenenfalls selbst ein- oder mehrfach, gleich oder verschieden substituiert mit Halogen oder Hydroxy.

Mehr bevorzugt steht $R^2$ für einen $C_2$-$C_6$-Alkyl-, $C_3$-$C_6$-Alkenyl- oder $C_3$-$C_6$-Alkinylrest oder einen $C_3$-$C_7$-Cycloalkylring, der gegebenenfalls ein- oder mehrfach substituiert ist mit Hydroxy, Halogen, $-CF_3$ und/oder $C_1$-$C_3$-Alkoxy.

Besonders bevorzugt steht $R^2$ für die Gruppe mit der Teilformel ($I$-$_{R}2$),

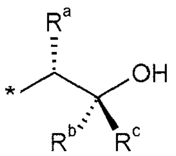

($I$-$_R2$)

in der

| | |
|---|---|
| $R^a$ | für eine Methyl-, Ethyl-, Propyl- oder Isopropylgruppe steht, und |
| $R^b$ und $R^c$ | unabhängig voneinander für Wasserstoff, eine Methyl- oder Ethylgruppe stehen. |

Formel (Ia) fasst diese Gruppe von Verbindungen zusammen.

Bevorzugt stehen $R^a$ und $R^b$ für eine Methylgruppe und $R^c$ für Wasserstoff oder eine Methylgruppe.

In der allgemeinen Formel (I) können $R^3$ und $R^4$ unabhängig voneinander stehen für: Wasserstoff und/oder einen $C_1$-$C_6$-Alkylrest, $C_2$-$C_6$-Alkenylrest, $C_3$-$C_8$-Cycloalkyl- und/oder Phenylring, einen Heterocyclylring mit 3 bis 8 Ringatomen und/oder einen monocyclischen Heteroarylring, gegebenenfalls mit Hydroxy, $-NR^5R^6$, Cyano, Halogen, $-CF_3$, $C_1$-$C_6$-Alkoxy und/oder $-OCF_3$ ein- oder mehrfach, gleich oder verschieden substituiert sind,
oder
$R^3$ und $R^4$ bilden gemeinsam mit dem Stickstoffatom einen 5- bis 7-gliedrigen Ring, der gegebenenfalls zusätzlich zum Stickstoffatom 1 oder 2 weitere Heteroatome enthält und der mit Hydroxy, $-NR^5R^6$, Cyano, Halogen, $-CF_3$, $C_1$-$C_6$-Alkoxy und/oder $-OCF_3$ ein- oder mehrfach, gleich oder verschieden substituiert sein kann.

Bevorzugt stehen $R^3$ und $R^4$ unabhängig voneinander für Wasserstoff und/oder einen $C_1$-$C_6$-Alkylrest, $C_3$-$C_8$-Cycloalkyl- und/oder Phenylring, gegebenenfalls mit Hydroxy, Cyano, Halogen, $-CF_3$, $C_1$-$C_6$-Alkoxy und/oder $-OCF_3$ ein- oder mehrfach, gleich oder verschieden substituiert.

In der allgemeinen Formel (I) können $R^5$ und $R^6$ unabhängig voneinander stehen für: Wasserstoff oder einen $C_1$-$C_6$-Alkylrest stehen, der gegebenenfalls mit Hydroxy, Cyano, Halogen, $-CF_3$, $C_1$-$C_6$-Alkoxy und/oder $-OCF_3$ ein- oder mehrfach, gleich oder verschieden substituiert ist.

Bevorzugt stehen $R^5$ und $R^6$ unabhängig voneinander für Wasserstoff und/oder einen $C_1$-$C_3$-Alkylrest.

Eine bevorzugte Untergruppe bilden Verbindungen der allgemeinen Formel (I),
in der

| | |
|---|---|
| $R^1$ | für einen $C_1$-$C_6$-Alkyl-, $C_2$-$C_6$-Alkenyl-, $C_2$-$C_6$-Alkinylrest oder einen $C_3$-$C_7$-Cycloalkyl oder Phenylring steht, jeweils gegebenenfalls mit Hydroxy, Cyano, Halogen, $-CF_3$, $C_1$-$C_6$-Alkoxy, $-OCF_3$ und/oder $C_1$-$C_6$-Alkyl ein- oder mehrfach, gleich oder verschieden substituiert, und |
| $R^2$ | für einen $C_1$-$C_{10}$-Alkyl-, $C_3$-$C_{10}$-Alkenyl- oder $C_3$-$C_{10}$-Alkinylrest oder einen $C_3$-$C_7$-Cycloalkylring steht, jeweils gegebenenfalls ein- oder mehrfach, gleich oder verschieden substituiert mit Halogen, Hydroxy, Cyano, $-CF_3$, $-OCF_3$, und/oder $C_1$-$C_6$-Alkoxy, $C_1$-$C_6$-Alkyl, jeweils gegebenenfalls selbst ein- oder mehrfach, gleich oder verschieden substituiert mit Halogen oder Hydroxy, |

sowie deren Salze, Diatereomere und Enantiomere.

Eine mehr bevorzugte Untergruppe bilden Verbindungen der allgemeinen Formel (I) in der

| | |
|---|---|
| $R^1$ | für einen $C_1$-$C_6$-Alkyl- oder $C_2$-$C_6$-Alkenylrest oder einen $C_3$-$C_7$- Cycloalkyl- oder Phenylring steht, jeweils gegebenenfalls mit Hydroxy, Cyano, Halogen und/oder $C_1$-$C_6$-Alkyl ein- oder mehrfach, gleich oder verschieden substituiert, und |

$R^2$ für einen $C_2$-$C_6$-Alkyl-, $C_3$-$C_6$-Alkenyl- oder $C_3$-$C_6$-Alkinylrest oder einen $C_3$-$C_7$-Cycloalkylring steht, der gegebenenfalls ein- oder mehrfach substituiert ist mit Hydroxy, Halogen, $-CF_3$ und/oder $C_1$-$C_3$- Alkoxy,

sowie deren Salze, Diatereomere und Enantiomere.

Eine besonders bevorzugte Untergruppe bilden Verbindungen der allgemeinen Formel (Ia)

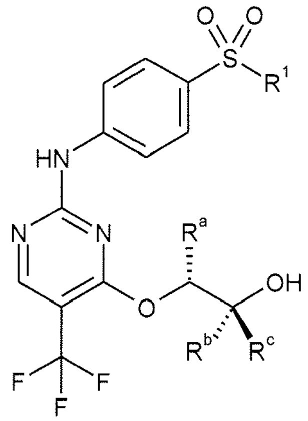

(Ia),

in der

| | |
|---|---|
| $R^1$ | für einen $C_1$-$C_4$-Alkyl- oder $C_2$-$C_4$-Alkenylrest oder einen $C_3$-$C_5$- Cycloalkyl- oder Phenylring steht, jeweils gegebenenfalls mit Hydroxy, Cyano, Halogen und/oder $C_1$-$C_3$-Alkyl ein- oder mehrfach, gleich oder verschieden substituiert, und |
| $R^a$ | für eine Methyl-, Ethyl-, Propyl- oder Isopropylgruppe steht, und |
| $R^b$ und $R^c$ | unabhängig voneinander für Wasserstoff, eine Methyl- oder Ethylgruppe stehen, |

sowie deren Salze, Diatereomere und Enantiomere.

Eine bevorzugte Untergruppe der Verbindungen der allgemeinen Formel (Ia) bildet die Gruppe von Verbindungen,
in der

| | |
|---|---|
| $R^1$ | für eine Methylgruppe oder für einen Cyclopropylring steht, und |
| $R^a$ und $R^b$ | für eine Methylgruppe stehen, und |
| $R^c$ | für Wasserstoff oder eine Methylgruppe steht, |

sowie deren Salze, Diatereomere und Enantiomere.

Die erfindungsgemäßen Verbindungen eignen sich zur Behandlung

- von Krebs, wie solide Tumore, Tumormetastasen, und Hämatologische Tumoren, insbesondere:

  Kopf- und Halstumore; Lungen- und Bronchialtumore; Gastrointestinaltumore wie z.B. Magenkarzinom, Kolorektales Karzinom, Pankreaskarzinom, Hepatozelluläres Karzinom; Endokin aktive Tumore; Mammakarzinome und Gynäkologische Tumore; Urogenitaltumore, wie z.B. Nierenzellkarzinom, Harnblasenkarzinom, Prostatakarzinom; Tumore der Haut; Sarkome; Leukämien und Lymphome.

- von viralen Erkrankungen, sowie
- von kardiovaskulären Erkrankungen wie Stenosen, Arteriosklerosen und Restenosen, Stent-induzierte Restenosen.

Die Formulierung der erfindungsgemäßen Verbindungen zu pharmazeutischen Präparaten erfolgt in an sich bekannter Weise, indem man den oder die Wirkstoffe mit den in der Galenik gebräuchlichen Hilfsstoffen in die gewünschte Applikationsform überführt.

Als Hilfsstoffe können dabei beispielsweise Trägersubstanzen, Füllstoffe, Sprengmittel, Bindemittel, Feuchthaltemittel, Gleitmittel, Ab- und Adsorptionsmittel, Verdünnungsmittel, Lösungsmittel, Cosolventien, Emulgatoren,

Lösungsvermittler, Geschmackskorrigentien, Färbemittel, Konservierungs-, Stabilisierungs-, Netzmittel, Salze zur Veränderung des osmotischen Drucks oder Puffer zum Einsatz kommen. Dabei ist auf Remington's Pharmaceutical Science, 15th ed. Mack Publishing Company, East Pennsylvania (1980) hinzuweisen.

Die pharmazeutischen Formulierungen können

in <u>fester</u> Form, zum Beispiel als Tabletten, Dragees, Pillen, Suppositorien, Kapseln, transdermale Systeme oder in <u>halbfester</u> Form , zum Beispiel als Salben, Cremes, Gele, Suppositorien, Emulsionen oder
in <u>flüssiger</u> Form, zum Beispiel als Lösungen, Tinkturen, Suspensionen oder Emulsionen vorliegen.

Hilfsstoffe im Sinne der Erfindung können beispielsweise Salze, Saccharide (Mono-, Di-, Tri-, Oligo-, und/oder Polysaccharide), Proteine, Aminosäuren, Peptide, Fette, Wachse, Öle, Kohlenwasserstoffe sowie deren Derivate sein, wobei die Hilfsstoffe natürlichen Ursprungs sein können oder synthetisch bzw. partial synthetisch gewonnen werden können.

Für die orale oder perorale Applikation kommen insbesondere Tabletten, Dragees, Kapseln, Pillen, Pulver, Granulate, Pastillen, Suspensionen, Emulsionen oder Lösungen in Frage.
Für die parenterale Applikation kommen insbesondere Suspensionen, Emulsionen und vor allem Lösungen in Frage.

**<u>Herstellung der erfindungsgemäßen Verbindungen</u>**

Die nachfolgenden Beispiele erläutern die Herstellung der erfindungsgemäßen Verbindungen, ohne den Umfang der beanspruchten Verbindungen auf diese Beispiele zu beschränken.

Die erfindungsgemäßen Verbindungen können hergestellt werden durch ein Verfahren, das durch folgende Schritte gekennzeichnet ist:

$a_1$) Funktionalisierung der 4-Position von 2,4-Dichlor-5-jod-pyrimidin (**1**) durch Umsetzung mit einem Alkohol der Formel (**2**) unter Bildung eines Intermediates der Formel (**3**)

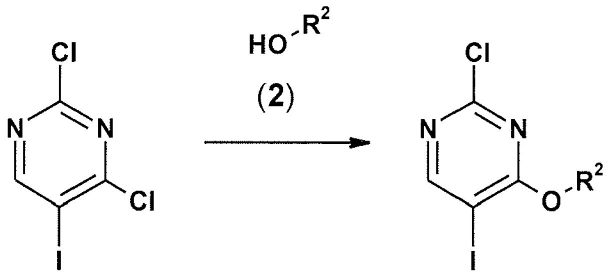

(**2**)

(**1**) (**3**)

und nachfolgende Umsetzung von Intermediat (**3**) unter Bildung des 5-$CF_3$ Intermediates (**4**)

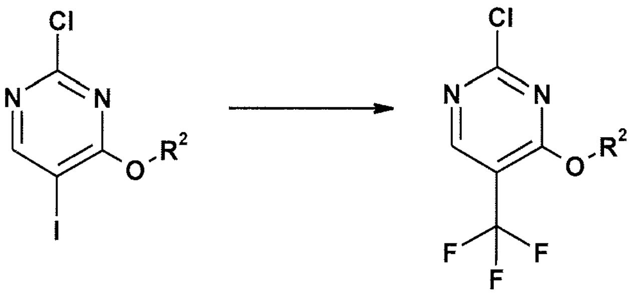

(**3**) (**4**)

oder alternativ

$a_2$) direkte Umsetzung von 2,4-Dichlor-5-trifluormethyl-pyrimidin (**5**) und einem Alkohol der Formel (**2**) unter Bildung des 5-$CF_3$ Intermediates (**4**).

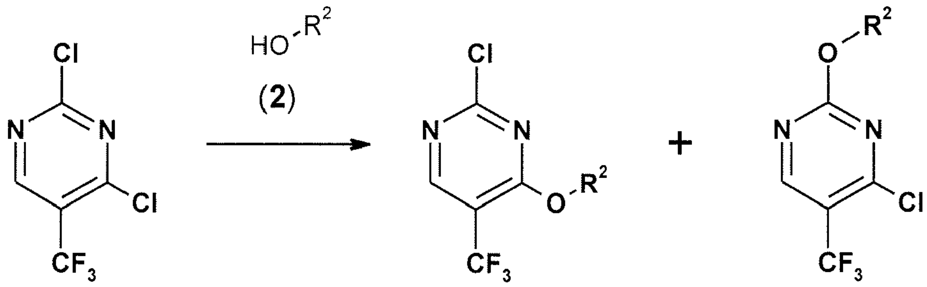

(**2**)

(**5**) (**4**) (**6**)

b) Oxidation eines Thioethers der Formel (**7**) zum Sulfon der Formel (**8**).

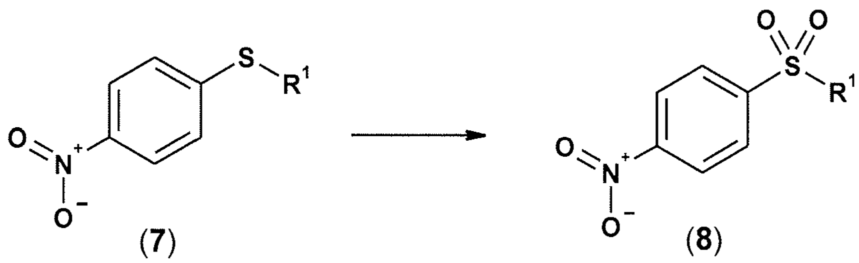

(**7**) (**8**)

c) Reduktion der Verbindung der Formel (**8**) zu einer Verbindung der Formel (**9**).

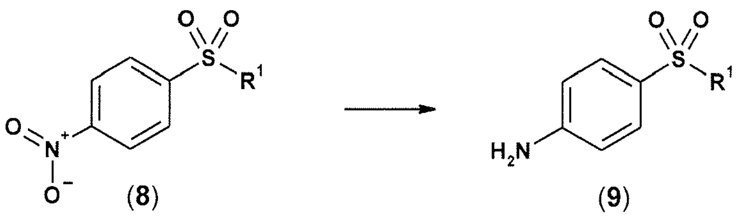

(**8**) (**9**)

d) Kupplung der Verbindungen der Formel (**4**) und (**9**).

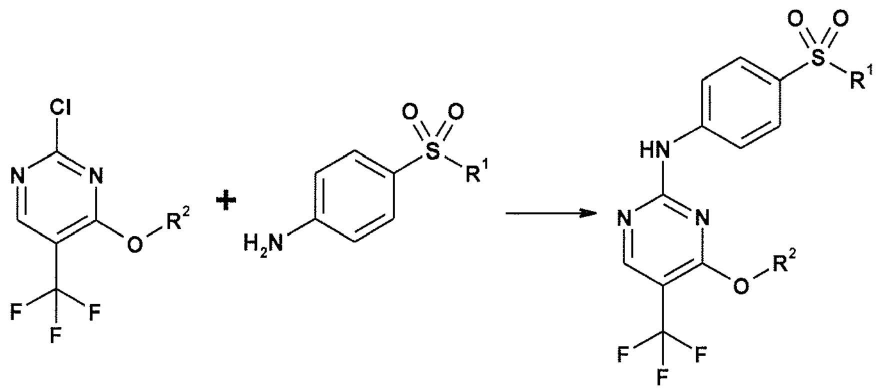

(4) (9) (I)

Gegebenfalls erfordert die Herstellung der erfindungsgemäßen Verbindungen das Einführen und anschließende Abspalten von Schutzgruppen (siehe z.B.: P.J. Kocienski, Protecting Groups, Georg Thieme Verlag Stuttgart, New York, 1994) wie z.B. in der Seitenkette der 4-Position.

Verfahrensschritt $a_1$)

Die Umsetzung von 2,4-Dichlor-5-jod-pyrimidin (**1**) mit einem Alkohol der Formel (**2**) unter basischen Bedingungen ermöglicht die Darstellung eines Produktes der Formel (**3**) (siehe z.B.: (a) U. Lücking et al, WO 2007/071455). Besonders geeignet für die Darstellung ist die beschriebene Verwendung von Natriumhydrid.

Für den Austausch eines Halogens gegen eine Trifluormethylgruppe in einem stickstoffhaltigen Heteroaromaten stehen prinzipiell verschiedene Methoden zur Verfügung (siehe z.B: a) G.E. Carr, R.D. Chambers, T.F. Holmes, J. Chem. Soc. Perkin Trans. 1, 1988, 921; b) F. Cottet, M. Schlosser, Eur. J. Org. Chem. 2002, 327; c) F.G. Njoroge et al, J. Med. Chem 1997, 40, 4290).
Besonders geeignet für den Austausch des Jods in der 5-Position des Pyrimidins (**3**) gegen eine $CF_3$-Gruppe unter Bildung einer Verbindung der Formel (**4**) ist die beschriebene Verwendung Kupfer(I)jodid, Kaliumfluorid und (Trifluormethyl)-trimethylsilan in N-Methyl-2-pyrrolidinon und THF.

Verfahrensschritt $a_2$)

Die Umsetzung von 2,4-Dichlor-5-trifluormethyl-pyrimidin (**5**) mit einem Alkohol der Formel (**2**) unter basischen Bedingungen ermöglicht die Darstellung der Produkte (**4**) und (**6**). Die Regioisomeren können in der Regel chromatographisch getrennt werden. (siehe z.B.: (a) T.M. Caldwell et al, WO 2006/081388, S. 50, Example 1, D). Besonders geeignet für die Darstellung ist die beschriebene Verwendung von Natriumhydrid.

Verfahrensschritt b)

Eine Verbindung der Formel (**7**) wird zum Sulfon der Formel (**8**) oxidiert. Für die Überführung eines Thioethers in ein Sulfon stehen zahlreiche Methoden zur Verfügung, z.B. unter Verwendung der Oxidationsmittel Wasserstoffperoxid oder Kaliumpermanganat. Besonders geeignet für die Darstellung von Verbindungen der Formel (**8**) ist die beschriebene Verwendung von meta-Chlorperbenzoesäure (MCPBA).

Verfahrensschritt c)

Für die anschließende Reduktion der aromatischen Nitrogruppe zu einer Verbindung der Formel (**9**) stehen prinzipiell eine Reihe von Reaktionsbedingungen zur Verfügung (siehe z.B.: R.C. Larock, Comprehensive Organic Transformations, VCH, New York, 1989, 411). Besonders geeignet ist beispielsweise die beschriebene Hydrierung unter Verwendung von Raney Nickel in THF.

Verfahrensschritt d)

Eine Verbindung der Formel (**4**) kann mit einem Anilin der Formel (**9**) zu einer Verbindung der Formel (I)

umgesetzt werden (siehe z.B.: (a) J. Bryant et al, WO 2004/048343).

**Allgemeine Hinweise**

Alle Reaktionen mit oxidations- oder hydrolyseempfindlichen Verbindungen wurden unter Argon und mit getrockneten Lösungsmitteln durchgeführt.
Die Substanzbenennung erfolgte unter Verwendung des Programms *Autonom 2000 Name*, welches in MDL ISIS Draw implementiert ist.

**Abkürzungen**

| Abkürzung | Bedeutung |
|---|---|
| Ac | Acetyl |
| Aloc | Allyloxycarbonyl |
| Boc | tert-Butyloxycarbonyl |
| BOM | Benzyloxymethyl |
| br | Broad |
| CI | Chemical ionisation |
| d | Dublett |
| dd | Dublett vom Dublett |
| DCM | Dichlormethan |
| DMF | *N*,*N*-Dimethylformamid |
| DMSO | Dimethylsulfoxid |
| ESI | Electrospray ionisation |
| HPLC | High performance liquid chromatography |
| m | Multiplett |
| M EM | (2-Methoxyethoxy)methyl |
| MOM | Methoxymethyl |
| MS | Mass spectrometry |
| MTM | Methylthiomethyl |
| NMP | N-Methyl-2-pyrrolidinon |
| NMR | Nuclear magnetic resonance spectroscopy : Chemische Verschiebung (δ) wird in ppm angegeben. |
| Pg | Protecting group umfassend Gruppen wie z.B. TMS, TES, TBDMS, TBDPS, TIPS, Benzyl, PMB, Trityl, Allyl, Aloc, MOM, MTM, MEM, BOM, SEM, THP. |
| PMB | p-Methoxybenzyl |
| q | Quartett |
| s | Singulett |
| SEM | β-(Trimethylsilyl)ethoxymethyl |
| TBDMS | Tert.-Butylsilyldimethyl |
| TBDPS | Tert.-Butylsilyldiphenyl |
| TEA | Triethylamin |
| TES | Triethylsilyl |

(fortgesetzt)

| Abkürzung | Bedeutung |
|---|---|
| THF | Tetrahydrofuran |
| THP | Tetrahydropyranyl |
| TIPS | Triisopropyl |
| TMS | Trimethylsilyl |
| tr | Triplett |

**Beispiel 1**

**(2R,3R)-3-[2-(4-Cyclopropansulfonyl-phenylamino)-5-trifluormethyl-pyrimidin-4-yloxy]-butan-2-ol**

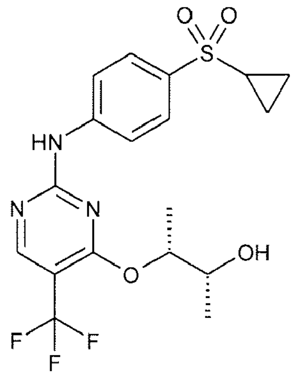

**1a) Herstellung der Zwischenprodukte**

**Verbindung 1.1**

**1-Cyclopropylsulfanyl-4-nitro-benzol**

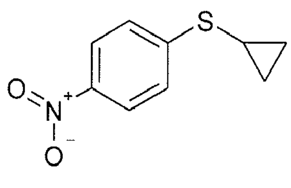

Eine 4%ige Lösung von 3,00 g (40,5 mmol) Cyclopropanthiol (Herstellung nach: E. Block et al, J. Am. Chem. Soc. 1992, 114, 3492) in THF / Diethylether (1:1) wurde portionsweise mit 1,78 g (44,6 mmol) Natriumhydrid (60%) versetzt und 30 Minuten bei Raumtemperatur gerührt. Anschließend erfolgte die portionsweise Zugabe von 6,00 g (38,7 mmol) 1-Fluor-4-nitro-benzol. Der Ansatz wurde 2 Stunden bei 40°C gerührt. Nach dem Abkühlen wurde der Ansatz in Wasser gegeben und mit Benzol extrahiert (3x). Die vereinten organischen Phasen wurden eingeengt und der Rückstand chromatographisch (Hexan / Essigester 95:5) gereinigt. Man erhielt 4,6 g (23,6 mmol; Ausbeute: 61 %) des Produktes. $^{1}$H-NMR (400 MHz, DMSO): δ= 8.12 (m, 2H), 7.54 (m, 2H), 2.35 (m, 1H), 1.16 (m, 2H), 0.61 (m, 2H).

**Verbindung 1.2**

**1-Cyclopropansulfonyl-4-nitro-benzol**

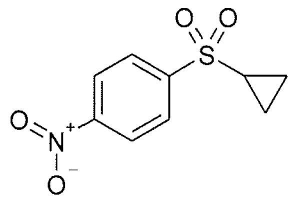

Eine Lösung von 1,00 g (5,12 mmol) 1-Cyclopropylsulfanyl-4-nitro-benzol in 120 ml DCM wurde bei 0°C mit 2,3 g meta-Chlorperbenzoesäure (max. 77%) versetzt und anschließend 4,5 Stunden bei Raumtemperatur gerührt. Der Ansatz wurde unter Rühren in eine gesättigte Natriumhydrogencarbonat-Lösung gegeben. Die organische Phase wurde über einem Whatman Filter filtriert und eingeengt. Der erhaltene Rückstand wurde chromatographisch (DCM / MeOH 95:5) gereinigt. Man erhielt 1,07 g (4,70 mmol; Ausbeute: 92%) des Produktes.
$^1$H-NMR (400 MHz, DMSO): δ = 8.41 (m, 2H), 8.15 (m, 2H), 2.98 (m, 1H), 1.11 (m, 4h).

**Verbindung 1.3**

**4-Cyclopropansulfonyl-phenylamin**

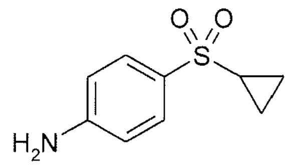

Eine Lösung von 1,60 g (7,0 mmol) 1-Cyclopropansulfonyl-4-nitro-benzol in 50 ml Ethanol und 50 ml THF wurde mit 3,2 g Raney-Nickel (50% wasserfeucht) versetzt und 1,5 Stunden unter Normaldruck bei 0°C hydriert. Der Ansatz wurde filtriert und eingeengt. Man erhielt 1,28 g (6,5 mmol; Ausbeute: 92 %) des Produktes.
$^1$H-NMR (400 MHz, DMSO): δ = 7.41 (m, 2H), 6.60 (m, 2H), 6.05 (br, 2H), 2.58 (m, 1H), 0.92 (m, 4H).
MS: 198 (ESI+).

**Verbindung 1.4**

**(2R,3R)-3-Benzyloxy-butan-2-ol**

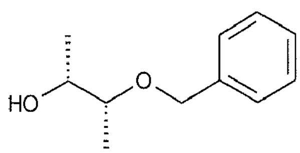

Eine Lösung von 4,0 g (44,4 mmol) (2R,3R)-Butan-2,3-diol in 300 ml THF wurde bei Raumtemperatur mit 5,0 g (44,6 mmol) Kalium-tert.-butylat versetzt und der Ansatz 15 Minuten refluxiert. Der Ansatz wurde auf ca. 50°C abgekühlt

und mit 5,3 ml (44,6 mmol) Benzylbromid versetzt. Man refluxierte für 3 Stunden, danach wurde über Nacht bei Raumtemperatur gerührt. Der Ansatz wurde mit Essigester und Natriumchlorid-Lösung verdünnt und anschließend mit 1 N Chlorwasserstoff-Lösung (1x) und Natriumchlorid-Lösung (2x) gewaschen. Die organische Phase wurde getrocknet ($Na_2SO_4$), filtriert und eingeengt. Der erhaltene Rückstand wurde chromatographisch (Hexan / Essigester 1:1) gereinigt. Man erhielt 3,4 g (18,9 mmol; Ausbeute: 43 %) des Produktes.
$^1$H-NMR (400 MHz, DMSO): δ = 7.35 (m, 4H), 7.28 (m, 1H), 4.52 (m, 3H), 3.67 (m, 1H), 3.37 (m, 1H), 1.05 (d, 3H), 1.01 (d, 3H).

**Verbindung 1.5**

**4-((1R,2R)-2-Benzyloxy-1-methyl-propoxy)-2-chlor-5-jod-pyrimidin**

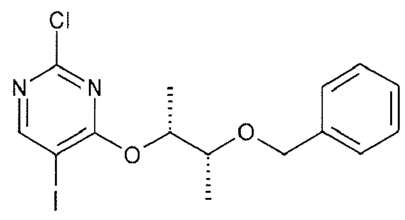

8,55 g (47,4 mmol) (2R,3R)-3-Benzyloxy-butan-2-ol in 56 ml Diethylether wurden bei 0°C unter Rühren portionsweise mit 2,07 g Natriumhydrid (55%) versetzt. Nach 10 Minuten wurde das Eisbad entfernt und weitere 3 Minuten bei Raumtemperatur gerührt. Die gebildete Suspension wurde bei 0°C zu einer Lösung von 6,52 g (23,7 mmol) 2,4-Dichlor-5-jod-pyrimidin in 65 ml Acetonitril gegeben. Der Ansatz wurde 4 Stunden bei 40°C gerührt und anschließend mit verdünnter Natriumchlorid-Lösung versetzt. Man extrahierte mit Essigester (2x). Die vereinten organischen Phasen wurden getrocknet ($Na_2SO_4$), filtriert und eingeengt. Der erhaltene Rückstand wurde chromatographisch (Hexan / Essigester 4:1) gereinigt. Man erhielt 4,12 g (9,8 mmol; Ausbeute: 41%) des Produktes.
$^1$H-NMR (400 MHz, DMSO): δ = 8.78 (s, 1H), 7.29 (m, 5H), 5.27 (m, 1H), 4.64 (d, 1H), 4.53 (d, 1H), 3.73 (m, 1H), 1.30 (d, 3H), 1.19 (d, 3H).

**Verbindung 1.6**

**4-((1R,2R)-2-Benzyloxy-1-methyl-propoxy)-2-chlor-5-trifluormethyl-pyrimidin**

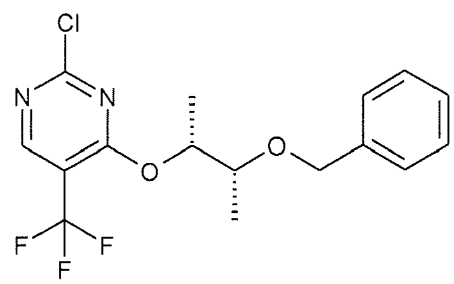

Eine Lösung von 4,66 g (11,1 mmol) 4-((1R,2R)-2-Benzyloxy-1-methyl-propoxy)-2-chlor-5-jod-pyrimidin in 15,8 ml NMP und 15,8 ml THF wurde bei Raumtemperatur unter Rühren mit 3,82 g (20,0 mmol) Kupfer(I)jodid, 0,97 g (16,7 mmol) Kaliumfluorid und 2,47 ml (16,7 mmol) (Trifluormethyl)-trimethylsilan versetzt. Der Ansatz wurde 5,5 Stunden bei 80°C gerührt. Nach dem Erkalten wurde der Ansatz in verdünnte Natriumchlorid-Lösung gegeben und mit Essigester extrahiert (2x). Die vereinten organischen Phasen wurden getrocknet ($Na_2SO_4$), filtriert und eingeengt. Der erhaltene Rückstand wurde chromatographisch (Hexan / Essigester 4:1) gereinigt. Man erhielt 2,17 g (6,0 mmol; Ausbeute: 54%)

des Produktes.
$^1$H-NMR (400 MHz, DMSO): δ = 8.81 (s, 1H), 7.21 (m, 5H), 5.40 (m, 1H), 4.57 (d, 1H), 4.42 (d, 1H), 3.70 (m, 1H), 1.28 (d, 3H), 1.13 (d, 3H).

Verbindung 1.6 wurde alternativ auch durch folgende Vorschrift hergestellt:

Eine Lösung von 5,00 g (23,0 mmol) 2,4-Dichlor-5-trifluormethyl-pyrimidin und 5,40 g (30,0 mmol) (2R,3R)-3-Benzyloxy-butan-2-ol in 60 ml Diethylether und 65 ml Acetonitril wurde bei 0°C mit 1,21 g (27,7 mmol) Natriumhydrd (55%ig), aufgeteilt in 3 Portionen, versetzt und anschließend 90 Minuten bei 15°C gerührt. Der Ansatz wurde mit verdünnter Natriumchlorid-Lösung versetzt und mit Essigester extrahiert (3x). Die vereinten organischen Phasen wurden getrocknet ($Na_2SO_4$), filtriert und eingeengt. Der erhaltene Rückstand wurde chromatographisch (Hexan / Essigester 95:5) gereinigt. Man erhielt 1,60 g (4,4 mmol; Ausbeute: 19%) des Produktes.

**Verbindung 1.7**

**[4-((1R,2R)-2-Benzyloxy-1-methyl-propoxy)-5-trifluormethyl-pyrimidin-2-yl]-(4-**cyclopropansulfonyl-phenyl)-amin

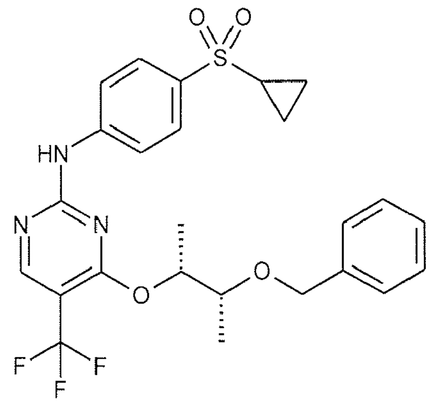

700 mg (1,94 mmol) 4-((1R,2R)-2-Benzyloxy-1-methyl-propoxy)-2-chlor-5-trifluormethyl-pyrimidin und 460 mg (2,33 mmol) 4-Cyclopropansulfonyl-phenylamin in 9,5 ml Acetonitril wurden mit 0,49 ml einer 4N Lösung von Chlorwasserstoff in Dioxan versetzt und 5,5 Stunden bei 80°C gerührt. Nach dem Abkühlen wurde der Ansatz mit Essigester verdünnt und mit gesättigter Natriumhydrogencarbonat-Lösung und gesättigter Natriumchlorid-Lösung gewaschen, getrocknet ($Na_2SO_4$), filtriert und eingeengt. Der erhaltene Rückstand wurde chromatographisch (DCM /Ethanol 95:5) gereinigt. Man erhielt 649 mg (1,24 mmol, Ausbeute: 64%) des Produktes.
$^1$H-NMR (400 MHz, DMSO): δ = 10.55 (s, 1H), 8.58 (s, 1H), 7.96 (m, 2H), 7.79 (m, 2H), 7.22 (m, 5H), 5.48 (m, 1H), 4.57 (d, 1H), 4.46 (d, 1H), 3.73 (m, 1H), 2.71 (m, 1H), 1.31 (d, 3H), 1.15 (d, 3H), 1.05 (m, 2H), 0.96 (m, 2H).
MS: 522 (ESI+)

**b) Herstellung des Endproduktes**

Eine Lösung von 541 mg (1,04 mmol) [4-((1R,2R)-2-Benzyloxy-1-methyl-propoxy)-5-trifluormethyl-pyrimidin-2-yl]-(4-cyclopropansulfonyl-phenyl)-amin in 110 ml Ethanol wurde mit 541 mg Palladium auf Kohle (10%ig) versetzt und unter Normaldruck bei Raumtemperatur für eine Stunde hydriert. Der Ansatz wurde filtriert und eingeengt. Der erhaltene Rückstand wurde chromatographisch (DCM / EtOH 98:2) gereinigt. Man erhielt 175 mg (0,41 mmol; Ausbeute: 39 %) des Produktes.
$^1$H-NMR (400 MHz, DMSO): δ = 10.54 (s, 1H), 8.56 (s, 1H), 7.95 (m, 2H), 7.79 (m, 2H), 5.28 (m, 1H), 4.86 (d, 1H), 3.83 (m, 1H), 2.76 (m, 1H), 1.26 (d, 3H), 1.01 (m, 7H).
MS: 432 (ESI+).

**Beispiel 2**

**(2R,3R)-3-[2-(4-Methansulfonyl-phenylamino)-5-trifluormethyl-pyrimidin-4-yloxy]-butan-2-ol**

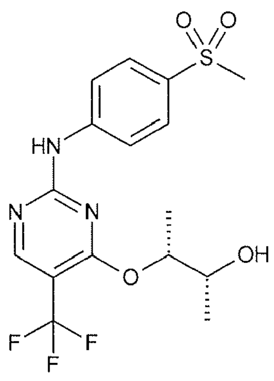

**2a) Herstellung der Zwischenprodukte**

**Verbindung 2.1**

**[4-((1R,2R)-2-Benzyloxy-1-methyl-propoxy)-5-trifluormethyl-pyrimidin-2-yl]-(4-**methansulfonyl-phenyl)-amin

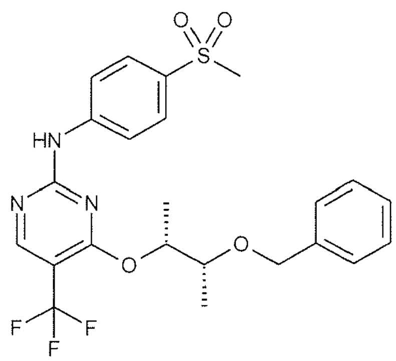

810 mg (2,25 mmol) 4-((1R,2R)-2-Benzyloxy-1-methyl-propoxy)-2-chlor-5-trifluormethyl-pyrimidin und 559 mg (2,69 mmol) 4-Methansulfonyl-phenylamin Hydrochlorid (Acros) in 11 ml Acetonitril wurden 16 Stunden bei 80°C gerührt. Nach dem Abkühlen wurde der Ansatz mit Essigester verdünnt und mit gesättigter Natriumhydrogencarbonat-Lösung und gesättigter NaCl-Lösung gewaschen, getrocknet ($Na_2SO_4$), filtriert und eingeengt. Der erhaltene Rückstand wurde chromatographisch (Hexan / Essigester 1:1) gereinigt. Man erhielt 770 mg (1,55 mmol; Ausbeute: 69%) des Produktes.
$^1$H-NMR (400 MHz, DMSO): $\delta$ = 10.55 (s, 1H), 8.58 (s, 1H), 7.95 (m, 2H), 7.83 (m, 2H), 7.22 (m, 5H), 5.48 (m, 1H), 4.57 (d, 1H), 4.46 (d, 1H), 3.72 (m, 1H), 3.12 (s, 3H), 1.31 (d, 3H), 1.15 (d, 3H).
MS: 496 (ESI+)

**b) Herstellung des Endproduktes**

Eine Lösung von 750 mg (1,51 mmol) [4-((1R,2R)-2-Benzyloxy-1-methyl-propoxy)-5-trifluormethyl-pyrimidin-2-yl]-(4-methansulfonyl-phenyl)-amin in 20 ml Ethanol wurde mit 152 mg Palladium auf Kohle (10%ig) versetzt und unter Normaldruck bei Raumtemperatur für 30 Minuten hydriert. Man versetzte erneut mit 152 mg Palladium auf Kohle (10%ig) und hydrierte für 1,5 Stunden. Weitere 152 mg Palladium auf Kohle (10%ig) wurden zugegeben und der Ansatz für 1 Stunde hydriert. Abschließend wurden nochmals 152 mg Palladium auf Kohle (10%ig) zugegeben und 15 Minuten

hydriert. Der Ansatz wurde filtriert und eingeengt. Der erhaltene Rückstand wurde chromatographisch (DCM / EtOH 95: 5) gereinigt. Man erhielt 512 mg (1,26 mmol; Ausbeute: 83 %) des Produktes.
$^1$H-NMR (400 MHz, DMSO): δ=10.55 (s, 1H), 8.57 (s, 1H), 7.96 (m, 2H), 7.83 (m, 2H), 5.27 (m, 1H), 4.86 (d, 1H), 3.82 (m, 1H), 3.14 (s, 3H), 1.25 (d, 3H), 1.07 (d, 3H). MS: 405 (EI+).

Beispiel 3

**(R)-3-[2-(4-Methansulfonyl-phenylamino)-5-trifluormethyl-pyrimidin-4-yloxy]-2-methyl-butan-2-ol**

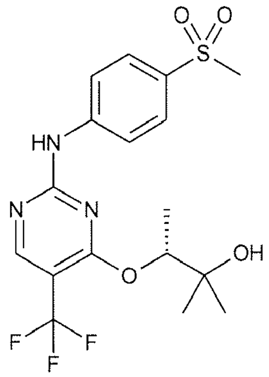

**3a) Herstellung der Zwischenprodukte**

**Verbindung 3.1.**

**(R)-2-Methyl-butan-2,3-diol**

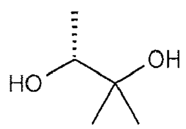

Eine Lösung von 10,0 g (95,1 mmol) (R)-(+)-Milchsäuremethylester in 20 ml THF wurde langsam zu 160 ml (480,0 mmol) einer eisgekühlten 3N Lösung von Methylmagnesiumchlorid in THF getropft. Der Ansatz wurde zunächst langsam auf Raumtemperatur erwärmt und anschließend 30 Minuten refluxiert. Nach dem Abkühlen wurde der Ansatz auf eine gesättigte Ammoniumchlorid-Lösung gegeben und mit Essigester extrahiert (3x). Die vereinten organischen Phasen wurden über einem Whatman Filter filtriert und eingeengt. Man erhielt 4,5 g (43,1 mmol) des Rohproduktes, das ohne weitere Reinigung eingesetzt wurde.
$^1$H-NMR (400 MHz, DMSO): δ = 4.21 (d, 1H), 3.93 (s, 1H), 3.29 (m, 1H), 0.97 (m, 9H).

Verbindung 3.2.

**(R)-3-(2-Chlor-5-jod-pyrimidin-4-yloxy)-2-methyl-butan-2-ol**

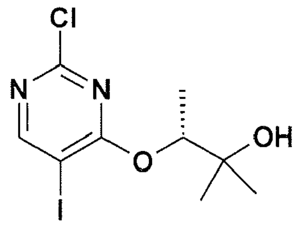

Eine Lösung von 4,40 g (42,3 mmol) (R)-2-Methyl-butan-2,3-diol in 83 ml Diethylether wurde unter Rühren bei 0°C portionsweise mit 1,84 g (42,3 mmol) Natriumhydrid (55%) versetzt und 10 Minuten gerührt. Man rührte weitere 3 Minuten bei Raumtemperatur und gab den Ansatz anschließend zu einer eisgekühlten Lösung von 9,68 g (35,2 mmol) 2,4-Dichlor-5-jod-pyrimidin in 97 ml Acetonitril. Der Ansatz wurde 4 Stunden bei 40°C gerührt und nach dem Erkalten mit Eis und gesättigter NaCl-Lösung versetzt. Man extrahierte mit Essigester (3x). Die vereinten organischen Phasen wurden getrocknet ($Na_2SO_4$), filtriert und eingeengt. Der erhaltene Rückstand wurde chromatographisch (Hexan / Essigester 4:1) gereinigt. Man erhielt 4,96 g (14,5 mmol; Ausbeute: 41%) des Produktes.
$^1$H-NMR (400 MHz, DMSO): δ = 8.73 (s, 1H), 4.96 (q, 1H), 4.62 (s, 1H), 1.21 (d, 3H), 1.13 (s, 6H).
ES: 343 (Cl+).

**Verbindung 3.3.**

**2-Chlor-4-[(R)-1,2-dimethyl-2-(tetrahydro-pyran-2-yloxy)-propoxy]-5-jod-**pyrimidin

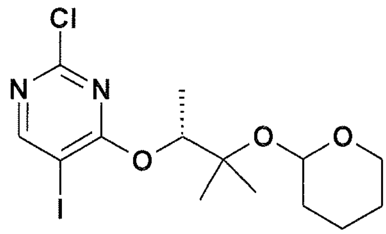

Eine Lösung von 4,96 g (14,5 mmol) (R)-3-(2-Chlor-5-jod-pyrimidin-4-yloxy)-2-methyl-butan-2-ol in 30 ml DCM wurde mit 2,64 ml (29,0 mmol) Dihydropyran und 0,36 g (1,5 mmol) Pyridiniumtosylat versetzt und 22 Stunden bei Raumtemperatur gerührt. Der Ansatz wurde mit DCM verdünnt und mit gesättigter Natriumhydrogencarbonat-Lösung gewaschen. Die organische Phase wurde getrocknet ($Na_2SO_4$), filtriert und eingeengt. Der erhaltene Rückstand wurde chromatographisch (Hexan / Essigester 4:1) gereinigt. Man erhielt 5,50 g (12,9 mmol; Ausbeute: 89%) des Diastereomerengemisches.
$^1$H-NMR (400 MHz, OMSO):δ = 8.75 (s, 1H), 8.74 (s, 1H), 5.15 (m, 2H), 4.91 (m, 2H), 3.70 (m, 2H), 3.30 (m, 2H), 1.31 (m, 30H).

**Verbindung 3.4.**

**2-Chlor-4-[(R)-1,2-dimethyl-2-(tetrahydro-pyran-2-yloxy)-propoxy]-5-trifluormethyl-pyrimidin**

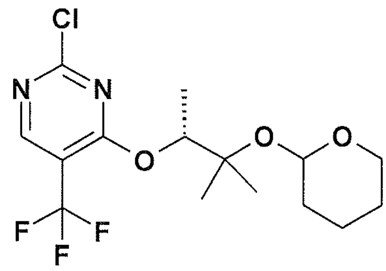

Eine Lösung von 1,00 g (2,34 mmol) 2-Chlor-4-[(R)-1,2-dimethyl-2-(tetrahydro-pyran-2-yloxy)-propoxy]-5-jod-pyrimidin in 3,3 ml NMP und 3,3 ml THF wurde bei Raumtemperatur mit 1,61 g (8,44 mmol) Kupfer(I)jodid, 0,41 g (7,03 mmol) Kaliumfluorid und 1,04 ml (7,03 mmol) (Trifluormethyl)-trimethylsilan versetzt. Der Ansatz wurde 2 Stunden bei 90°C gerührt. Nach dem Erkalten wurde der Ansatz in verdünnte Natriumchlorid-Lösung gegeben und mit Essigester extrahiert (3x). Die vereinten organischen Phasen wurden getrocknet ($Na_2SO_4$), filtriert und eingeengt. Der erhaltene Rückstand wurde chromatographisch (Hexan / Essigester 4:1) gereinigt. Man erhielt 0,53 g (1,43 mmol; Ausbeute: 61%) des Produktes.
$^1$H-NMR (400 MHz, DMSO): $\delta$ = 8.84 (s, 1H), 5.32 (m, 1H), 4.85 (m, 1H), 3.68 (m, 1H), 3.30 (m, 1H), 1.31 (m, 15H).

**b) Herstellung des Endproduktes**

100 mg (0,27 mmol) 2-Chlor-4-[(R)-1,2-dimethyl-2-(tetrahydro-pyran-2-yloxy)-propoxy]-5-trifluormethyl-pyrimidin und 37 mg (0,22 mmol) 4-Methansulfonyl-phenylamin in 2,5 ml Ethanol wurden 150 Minuten bei 70°C gerührt. Der Ansatz wurde zur Trockene eingeengt, mit 3,1 ml Ethanol aufgenommen und mit 12 mg (0,05 mmol) Pyridiniumtosylat versetzt. Der Ansatz wurde 4 Stunden bei 45°C gerührt. Nach dem Erkalten wurde der Ansatz mit verdünnter Natriumhydrogencarbonat-Lösung versetzt und mit Essigester extrahiert (3x). Die vereinten organischen Phasen wurden getrocknet ($Na_2SO_4$), filtriert und eingeengt. Der erhaltene Rückstand wurde chromatographisch (DCM / Ethanol 95:5) gereinigt. Man erhielt 42 mg (0,10 mmol; Ausbeute: 45%) des Produktes.
$^1$H-NMR (400 MHz, DMSO): $\delta$ = 10.55 (s, 1H), 8.57 (s, 1H), 7.96 (m, 2H), 7.84 (m, 2H), 5.14 (q, 1H), 4.66 (s, 1H), 3.14 (s, 3H), 1.28 (d, 3H), 1.12 (s, 6H).
MS: 419 (EI+)

**Beispiel 4**

**(R)-3-[2-(4-Cyclopropansulfonyl-phenylamino)-5-trifluormethyl-pyrimidin-4-yloxy]-2-methyl-butan-2-ol**

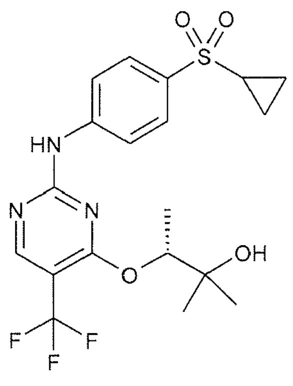

**Herstellung des Endproduktes**

200 mg (0,54 mmol) 2-Chlor-4-[(R)-1,2-dimethyl-2-(tetrahydro-pyran-2-yloxy)-propoxy]-5-trifluormethyl-pyrimidin und 64 mg (0,33 mmol) 4-Cyclopropansulfonyl-phenylamin in 5,0 ml Ethanol wurden 210 Minuten bei 70°C gerührt. Der Ansatz wurde erneut mit 100 mg (0,27 mmol) 2-Chlor-4-[(R)-1,2-dimethyl-2-(tetrahydropyran-2-yloxy)-propoxy]-5-trifluormethyl-pyrimidin versetzt und weitere 210 Minuten bei 70°C gerührt. Der Ansatz wurde zur Trockene eingeengt und der erhaltene Rückstand wurde mittels HPLC gereinigt. Man erhielt 91 mg (0,20 mmol; Ausbeute: 61 %) des Produktes.

| | | |
|---|---|---|
| Säule: | XBridge C18 5µ 100x30 mm | |
| Eluent A: | $H_2O$ / 0.2% $NH_3$ | |
| Eluent B: | Acetonitril | |
| Gradient: | 0 min | 50%A50%B |
| | 1.00 min | 50%A 50%B |
| | 7.50 min | 20%A 80%B |
| | 7.52 min | 1%A 99%B |
| | 10.00 min | 1%A 99%B |
| Fluß: | 50,0 mL/min | |
| Detektor: | DAD scan range 210-400 nm | |
| | MS ESI+, ESI-, scan range 160-1000 m/z | |
| Temperatur: | Raumtemperatur | |
| Retentionszeit: | 4,6-5,5 min | |

$^1$H-NMR (400 MHz, DMSO): 10.56 (br, 1H), 8.57 (s, 1H), 7.96 (m, 2H), 7.80 (m, 2H), 5.14 (q, 1H), 4.66 (s, 1H), 2.77 (m, 1H), 1.28 (d, 3H), 1.12 (m, 6H), 1.07 (m, 2H), 0.97 (m, 2H).
MS: 445 (EI+)

**Beispiel 5**

**(2R,3R)-3-[2-(4-Benzolsulfonyl-phenylamino)-5-trifluormethyl-pyrimidin-4-yloxy]-butan-2-ol**

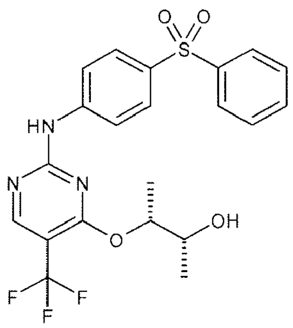

**5a) Herstellung der Zwischenprodukte**

**Verbindung 5.1**

**(4-Benzolsulfonyl-phenyl)-[4-((1R,2R)-2-benzyloxy-1-methyl-propoxy)-5-trifluormethyl-pyrimidin-2-yl]-amin**

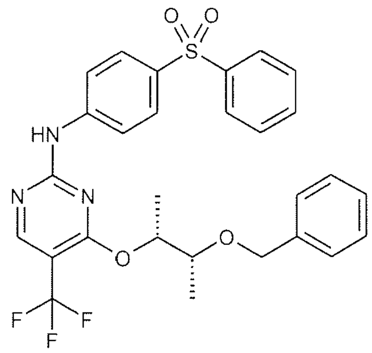

110 mg (0,30 mmol) 4-((1R,2R)-2-Benzyloxy-1-methyl-propoxy)-2-chlor-5-trifluormethyl-pyrimidin und 85 mg (0,37 mmol) 4-Benzolsulfonyl-phenylamin in 1,5 ml Acetonitril wurden mit 0,08 ml einer 4N Lösung von Chlorwasserstoff in Dioxan versetzt und 5 Stunden bei 80°C gerührt. Der Ansatz wurde einrotiert und der erhaltene Rückstand mittels HPLC gereinigt. Man erhielt 121 mg (0,22 mmol, Ausbeute: 71 %) des Produktes.

| | | | |
|---|---|---|---|
| System: | Waters Autopurification | | |
| Säule: | XBridge C18 5µ 100x30 mm | | |
| Eluent A: | $H_2O$ / 0.1 % HCOOH | | |
| Eluent B: | Acetonitril | | |
| Gradient: | 0 min | 99%A | 1 %B |
| | 1.00 min | 99%A | 1%B |
| | 7.50 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |

$^1$H-NMR (400 MHz, DMSO): δ = 10.56 (s, 1H), 8.56 (s, 1H), 7.89 (m, 6H), 7.58 (m, 3H), 7.22 (m, 5H), 5.44 (m, 1H), 4.55 (d, 1H), 4.44 (d, 1H), 3.71 (m, 1H), 1.29 (d, 3H), 1.14 (d, 3H).

**b) Herstellung des Endproduktes**

Eine Lösung von 116 mg (0,21 mmol) (4-Benzolsulfonyl-phenyl)-[4-((1R,2R)-2-benzyloxy-1-methyl-propoxy)-5-trifluormethyl-pyrimidin-2-yl]-amin in 5 ml Ethanol wurde 2,5 Stunden bei Raumtemperatur unter einer Wasserstoffatmosphäre hydriert. Dabei wurde der Ansatz 6x mit jeweils 50 mg Portionen von Palladium auf Kohle (10%ig) versetzt. Der Ansatz wurde filtriert und eingeengt. Man erhielt 63 mg (0,13 mmol; Ausbeute: 65 %) des Produktes.
$^1$H-NMR (400 MHz, DMSO): δ = 10.55 (s, 1H), 8.55 (s, 1H), 7.89 (m, 6H), 7.59 (m, 3H), 5.25 (m, 1H), 4.86 (d, 1H), 3.80 (m, 1H), 1.24 (d, 3H), 1.06 (d, 3H). MS: 468 (ESI+)

**Beispiel 6**

**(2R,3R)-3-{2-[4-(Difluor-methansulfonyl)-phenylamino]-5-trifluormethyl-pyrimidin-4-yloxy}-butan-2-ol**

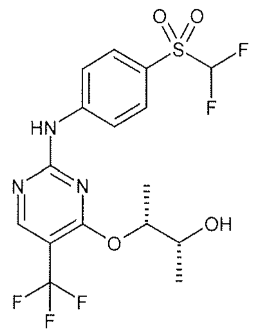

**6a) Herstellung der Zwischenprodukte**

**Verbindung 6.1**

**[4-((1R,2R)-2-Benzyloxy-1-methyl-propoxy)-5-trifluormethyl-pyrimidin-2-yl]-[4-**(difluor-methansulfonyl)-phenyl]-amin

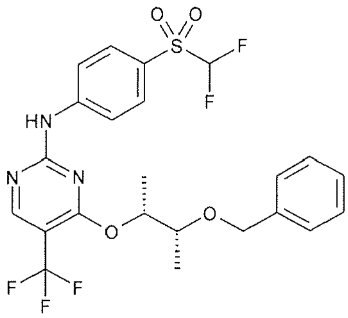

100 mg (0,28 mmol) 4-((1R,2R)-2-Benzyloxy-1-methyl-propoxy)-2-chlor-5-trifluormethyl-pyrimidin und 69 mg (0,33 mmol) 4-(Difluor-methansulfonyl)-phenylamin in 1,4 ml Acetonitril wurden mit 0,07 ml einer 4N Lösung von Chlorwasserstoff in Dioxan versetzt und 3 Stunden bei 80°C gerührt. Der Ansatz wurde einrotiert und der erhaltene Rückstand mittels HPLC gereinigt. Man erhielt 104 mg (0,20 mmol; Ausbeute: 71%) des Produktes.

| | | | |
|---|---|---|---|
| System: | Waters Autopurification | | |
| Säule: | XBridge C18 5µ 100x30 mm | | |
| Eluent A: | $H_2O$ / 0.1 % HCOOH | | |
| Eluent B: | Acetonitril | | |
| Gradient: | 0 min | 99%A | 1%B |
| | 1.00 min | 99%A | 1%B |
| | 7.50 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |

$^1$H-NMR (400 MHz, DMSO): δ = 10.77 (s, 1H), 8.62 (s, 1H), 8.07 (m, 2H), 7.88 (m, 2H), 7.22 (m, 6H), 5.47 (m, 1H), 4.57 (d, 1H), 4.45 (d, 1H), 3.72 (m, 1H), 1.31 (d, 3H), 1.15 (d, 3H).

**b) Herstellung des Endproduktes**

Eine Lösung von 100 mg (0,19 mmol) [4-((1R,2R)-2-Benzyloxy-1-methyl-propoxy)-5-trifluormethyl-pyrimidin-2-yl]-[4-(difluor-methansulfonyl)-phenyl]-amin in 5 ml Ethanol wurde 1,5 Stunden bei Raumtemperatur unter einer Wasserstoffatmosphäre hydriert. Dabei wurde der Ansatz 3x mit jeweils 100 mg Portionen von Palladium auf Kohle (10%ig) versetzt. Der Ansatz wurde filtriert und eingeengt. Man erhielt 45 mg (0,10 mmol; Ausbeute: 54 %) des Produktes. $^1$H-NMR (400 MHz, DMSO): $\delta$ =10.77 (s, 1H), 8.61 (s, 1H), 8.08 (m, 2H), 7.88 (m, 2H), 7.20 (tr, 1H), 5.29 (m, 1H), 4.88 (d, 1H), 3.84 (m, 1H), 1.26 (d, 3H), 1.07 (d, 3H). MS: 442 (ESI+)

Beispiel 7

**(2R,3R)-3-[2-(4-Cyclopentansulfonyl-phenylamino)-5-trifluormethyl-pyrimidin-4-yloxy]-butan-2-ol**

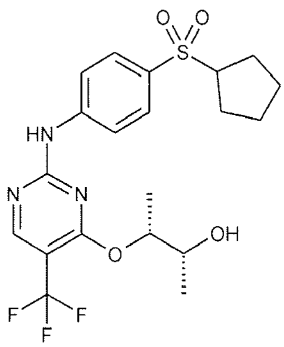

**7a) Herstellung der Zwischenprodukte**

**Verbindung 7.1**

**[4-((1R,2R)-2-Benzyloxy-1-methyl-propoxy)-5-trifluormethyl-pyrimidin-2-yl]-(4-cyclopentansulfonyl-phenyl)-amin**

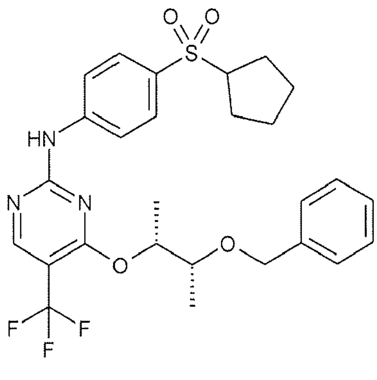

100 mg (0,28 mmol) 4-((1R,2R)-2-Benzyloxy-1-methyl-propoxy)-2-chlor-5-trifluormethyl-pyrimidin und 75 mg (0,33 mmol) 4-Cyclopentansulfonyl-phenylamin in 1,4 ml Acetonitril wurden mit 0,07 ml einer 4N Lösung von Chlorwasserstoff in Dioxan versetzt und 5 Stunden bei 80°C gerührt. Der Ansatz wurde einrotiert und der erhaltene Rückstand mittels HPLC gereinigt. Man erhielt 118 mg (0,21 mmol, Ausbeute: 77%) des Produktes.

| | | | |
|---|---|---|---|
| System: | Waters Autopurification | | |
| Säule: | XBridge C18 5µ 100x30 mm | | |
| Eluent A: | $H_2O$ / 0.1 % HCOOH | | |
| Eluent B: | Acetonitril | | |
| Gradient: | 0 min | 99%A | 1%B |
| | 1.00 min | 99%A | 1%B |
| | 7.50 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |

$^1$H-NMR (400 MHz, DMSO): δ = 10.57 (s, 1H), 8.58 (s, 1H), 7.96 (m, 2H), 7.77 (m, 2H), 7.22 (m, 5H), 5.48 (m, 1H), 4.56 (d, 1H), 4.46 (d, 1H), 3.72 (m, 1H), 3.63 (m, 1H), 1.76 (m, 4H), 1.51 (m, 4H), 1.30 (d, 3H), 1.14 (d, 3H).

**b) Herstellung des Endproduktes**

Eine Lösung von 112 mg (0,20 mmol) [4-((1R,2R)-2-Benzyloxy-1-methyl-propoxy)-5-trifluormethyl-pyrimidin-2-yl]-(4-cyclopentansulfonyl-phenyl)-amin in 5 ml Ethanol wurde 2,5 Stunden bei Raumtemperatur unter einer Wasserstoffatmosphäre hydriert. Dabei wurde der Ansatz 5x mit jeweils 100 mg Portionen von Palladium auf Kohle (10%ig) versetzt. Der Ansatz wurde filtriert und eingeengt. Man erhielt 75 mg (0,16 mmol; Ausbeute: 80 %) des Produktes.
$^1$H-NMR (400 MHz, DMSO): δ=10.58 (s, 1H), 8.57 (s, 1H), 7.97 (m, 2H), 7.78 (m, 2H), 5.28 (m, 1H), 4.88 (d, 1H), 3.83 (m, 1H), 3.68 (m, 1H), 1.76 (m, 4H), 1.55 (m, 4H), 1.25 (d, 3H), 1.07 (d, 3H).
MS: 460 (ESI+)

**Beispiel 8**

**2R,3R)-3-{2-[4-(Prop-2-en-1-sulfonyl)-phenylamino]-5-trifluormethyl-pyrimidin-4-yloxy}-butan-2-ol**

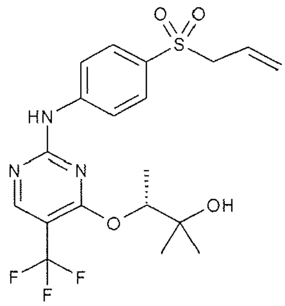

**Herstellung des Endproduktes**

104 mg (0,28 mmol) 2-Chlor-4-[(R)-1,2-dimethyl-2-(tetrahydro-pyran-2-yloxy)-propoxy]-5-trifluormethyl-pyrimidin und 33 mg (0,17 mmol) 4-(Prop-2-en-1-sulfonyl)-phenylamin in 2,6 ml Ethanol wurden 10 Stunden bei 70°C gerührt. Der Ansatz wurde zur Trockene eingeengt und der Rückstand mittels HPLC gereinigt. Man erhielt 12 mg (0,03 mmol; Ausbeute: 10%) des Produktes.

| | |
|---|---|
| System: | Waters Autopurification |
| Säule: | XBridge C18 5µ 100x30 mm |
| Eluent A: | $H_2O$ / 0.1 % HCOOH |

(fortgesetzt)

| | | | |
|---|---|---|---|
| Eluent B: | Acetonitril | | |
| Gradient: | 0 min | 99%A | 1%B |
| | 1.00 min | 99%A | 1%B |
| | 7.50 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |

$^1$H-NMR (400 MHz, DMSO): δ = 10.57 (s, 1H), 8.58 (s, 1H), 7.95 (m, 2H), 7.76 (m, 2H), 5.64 (m, 1H), 5.25 (m, 1H), 5.17 (m, 2H), 4.67 (s, 1H), 4.04 (d, 2H), 1.28 (d, 3H), 1.12 (s, 6H).

**Beispiel 9**

**(2R,3R)-3-{2-[4-(Propan-2-sulfonyl)-phenylamino]-5-trifluormethyl-pyrimidin-4-yloxy}-butan-2-ol**

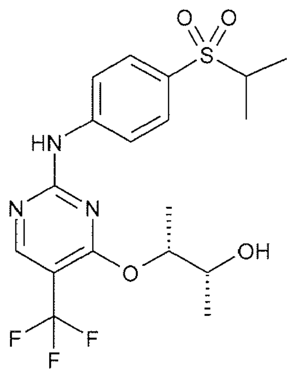

**9a) Herstellung der Zwischenprodukte**

**Verbindung 9.1**

**[4-((1R,2R)-2-Benzyloxy-1-methyl-propoxy)-5-trifluormethyl-pyrimidin-2-yl]-[4-(propan-2-sulfonyl)-phenyl]-amin**

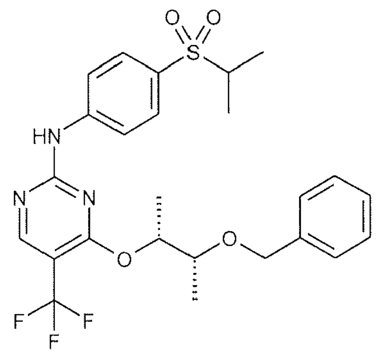

103 mg (0,29 mmol) 4-((1R,2R)-2-Benzyloxy-1-methyl-propoxy)-2-chlor-5-trifluormethyl-pyrimidin und 68 mg (0,34 mmol) 4-(Propan-2-sulfonyl)-phenylamin in 1,4 ml Acetonitril wurden mit 0,07 ml einer 4N Lösung von Chlorwasserstoff in Dioxan versetzt und 5 Stunden bei 80°C gerührt. Der Ansatz wurde einrotiert und der erhaltene Rückstand mittels HPLC gereinigt. Man erhielt 123 mg (0,22 mmol, Ausbeute: 82%) des Produktes.

| | | | |
|---|---|---|---|
| System: | Waters Autopurification | | |
| Säule: | XBridge C18 5µ 100x30 mm | | |
| Eluent A: | $H_2O$ / 0.1% HCOOH | | |
| Eluent B: | Acetonitril | | |
| Gradient: | 0 min | 99%A | 1%B |
| | 1.00 min | 99%A | 1%B |
| | 7.50 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |

$^1$H-NMR (400 MHz, DMSO): δ = 10.59 (s, 1H), 8.58 (s, 1H), 7.96 (m, 2H), 7.75 (m, 2H), 7.22 (m, 5H), 5.48 (m, 1H), 4.55 (d, 1H), 4.45 (d, 1H), 3.72 (m, 1H), 3.27 (m, 1H), 1.30 (d, 3H), 1.14 (d, 3H), 1.09 (d, 6H).

**b) Herstellung des Endproduktes**

Eine Lösung von 118 mg (0,23 mmol) [4-((1R,2R)-2-Benzyloxy-1-methyl-propoxy)-5-trifluormethyl-pyrimidin-2-yl]-[4-(propan-2-sulfonyl)-phenyl]-amin in 5 ml Ethanol wurde 1,5 Stunden bei Raumtemperatur unter einer Wasserstoffatmosphäre hydriert. Dabei wurde der Ansatz 4x mit jeweils 50 mg Portionen von Palladium auf Kohle (10%ig) versetzt. Der Ansatz wurde filtriert und eingeengt. Man erhielt 43 mg (0,10 mmol; Ausbeute: 44 %) des Produktes.
$^1$H-NMR (400 MHz, DMSO): δ= 10.58 (s, 1H), 8.57 (s, 1H), 7.98 (m, 2H), 7.76 (m, 2H), 5.27 (m, 1H), 4.87 (d, 1H), 3.81 (m, 1H), 3.31 (m, 1H), 1.25 (d, 3H), 1.11 (d, 6H), 1.06 (d, 3H).
MS: 434 (ESI+)

**Beispiel 10**

**(2R,3R)-3-{2-[4-(Prop-2-yne-1-sulfonyl)-phenylamino]-5-trifluormethyl-pyrimidin-4-yloxy}-butan-2-ol**

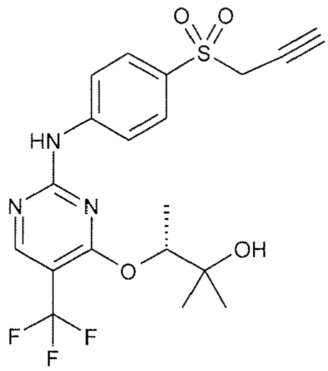

**Herstellung des Endproduktes**

150 mg (0,41 mmol) 2-Chlor-4-[(R)-1,2-dimethyl-2-(tetrahydro-pyran-2-yloxy)-propoxy]-5-trifluormethyl-pyrimidin und 47 mg (0,24 mmol) 4-(Prop-2-in-1-sulfonyl)-phenylamin in 3,6 ml Ethanol wurden 200 Minuten bei 70°C gerührt. Der Ansatz wurde zur Trockene eingeengt. Man erhielt ca. 147 mg des Rohproduktes von dem 60 mg mittels HPLC gereinigt wurden. Man erhielt 31 mg (0,07 mmol) des Produktes.

| | | | |
|---|---|---|---|
| System: | Waters Autopurification | | |
| Säule: | XBridge C18 5µ 100x30 mm | | |
| Eluent A: | $H_2O$ / 0.1 % HCOOH | | |
| Eluent B: | Acetonitril | | |
| Gradient: | 0 min | 99%A | 1%B |
| | 1.00 min | 99%A | 1%B |
| | 7.50 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |

$^1$H-NMR (400 MHz, DMSO): δ = 10.60 (s, 1H), 8.58 (s, 1H), 7.98 (m, 2H), 7.83 (m, 2H), 5.14 (q, 1H), 4.67 (s, 1H), 4.45 (d, 2H), 3.38 (tr, 1H), 1.28 (d, 3H), 1.10 (m, 6H). MS: 444 (ESI+)

**Beispiel 11**

**(2R,3R)-3-{2-[4-(2-Hydroxy-ethansulfonyl)-phenylamino]-5-trifluormethyl-pyrimidin-4-yloxy}-butan-2-ol**

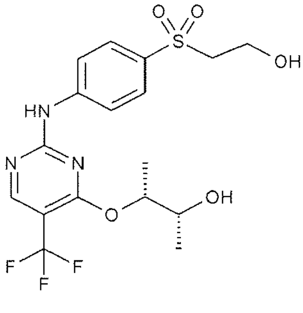

**11a) Herstellung der Zwischenprodukte**

**Verbindung 11.1**

**2-{4-[4-((1R,2R)-2-Benzyloxy-1-methyl-propoxy)-5-trifluormethyl-pyrimidin-2-ylamino]-benzolsulfonyl}-ethanol**

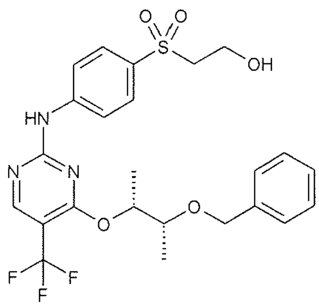

102 mg (0,28 mmol) 4-((1R,2R)-2-Benzyloxy-1-methyl-propoxy)-2-chlor-5-trifluormethyl-pyrimidin und 68 mg (0,34 mmol) 2-(4-Amino-benzolsulfonyl)-ethanol in 1,4 ml Acetonitril wurden mit 0,07 ml einer 4N Lösung von Chlorwasserstoff in Dioxan versetzt und 4 Stunden bei 80°C gerührt. Der Ansatz wurde einrotiert und der erhaltene Rückstand mittels HPLC gereinigt. Man erhielt 72 mg (0,14 mmol, Ausbeute: 48%) des Produktes.

| | | | |
|---|---|---|---|
| System: | Waters Autopurification | | |
| Säule: | XBridge C18 5µ 100x30 mm | | |
| Eluent A: | $H_2O$ / 0.1 % HCOOH | | |
| Eluent B: | Acetonitril | | |
| Gradient: | 0 min | 99%A | 1%B |
| | 1.00 min | 99%A | 1%B |
| | 7.50 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |

$^1$H-NMR (400 MHz, DMSO): δ = 10.56 (s, 1H), 8.58 (s, 1H), 7.94 (m, 2H), 7.79 (m, 2H), 7.22 (m, 5H), 5.46 (m, 1H), 4.83 (tr, 1H), 4.56 (d, 1H), 4.46 (d, 1H), 3.72 (m, 1H), 3.62 (m, 2H), 3.35 (m, 2H), 1.30 (d, 3H), 1.15 (d, 3H).

**b) Herstellung des Endproduktes**

Eine Lösung von 68 mg (0,13 mmol) (2-{4-[4-((1R,2R)-2-Benzyloxy-1-methyl-propoxy)-5-trifluormethyl-pyrimidin-2-ylamino]-benzolsulfonyl}-ethanol in 5 ml Ethanol wurde eine Stunden bei Raumtemperatur unter einer Wasserstoffatmosphäre hydriert. Dabei wurde der Ansatz mit jeweils 68 mg von Palladium auf Kohle (10%ig) versetzt. Der Ansatz wurde filtriert und eingeengt. Man erhielt 28 mg (0,06 mmol; Ausbeute: 50 %) des Produktes.
$^1$H-NMR (400 MHz, DMSO): δ = 10.55 (s, 1H), 8.57 (s, 1H), 7.95 (m, 2H), 7.79 (m, 2H), 5.27 (m, 1H), 4.87 (d, 1H), 4.84 (tr, 1H), 3.82 (m, 1H), 3.63 (m, 2H), 3.36 (m, 2H), 1.25 (d, 3H), 1.07 (d, 3H).
MS: 436 (ESI+)

**Beispiel 12**

**Assay 1: CDK1/CycB Kinase Assay**

Rekombinante CDK1- und CycB-GST-Fusionsproteine, gereinigt aus Bakulovirus-infizierten Insektenzellen (Sf9), wurden von ProQinase GmbH, Freiburg, gekauft. Das als Kinase-Substrat verwendet Histon IIIS ist über die Fa. Sigma käuflich zu erwerben.
CDK1/CycB (200 ng/Messpunkt) wurde für 10 min bei 22°C in Anwesenheit verschiedener Konzentrationen an Testsubstanzen (0 μM, sowie innerhalb des Bereiches 0,001 - 10 μM) in Assaypuffer [50 mM Tris/HCl pH8,0; 10 mM $MgCl_2$; 0,1 mM Na ortho-Vanadat; 1,0 mM Dithiothreitol; 0,5 μM Adenosintrisphosphat (ATP); 10 μg/Messpunkt Histon IIIS; 0,2 μCi/Messpunkt $^{33}$P-gamma ATP; 0,05% NP40; 1,25% Dimethylsulfoxid] inkubiert. Die Reaktion wurde durch Zugabe von EDTA-Lösung (250 mM; pH8,0; 15 μl/Messpunkt) gestoppt.
Von jedem Reaktionsansatz wurden 15 μl auf P30 Filterstreifen (Fa. Wallac) aufgetragen, und nicht-eingebautes $^{33}$P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLex™ A, Fa. Wallac) bedeckt und für 1 Stunde bei 90°C eingebrannt. Die Menge an eingebautem $^{33}$P (Substratphosphorylierung) wurde durch Szintillationsmessung in einem Gamma-Strahlungsmessgerät (Wallac) bestimmt. Die Messdaten wurden normalisiert auf 0% Inhibition (Enzymreaktion ohne Inhibitor) und 100% Inhibition (alle Assaykomponenten außer Enzym). Die Bestimmung der IC50 Werte erfolgte mittels eines 4-Parameter Fits unter Benutzung firmeneigener Software.

**Assay 2: CDK2/CycE Kinase Assay**

Rekombinante CDK2- und CycE-GST-Fusionsproteine, gereinigt aus Bakulovirus-infizierten Insektenzellen (Sf9), wurden von ProQinase GmbH, Freiburg, gekauft. Histon IIIS, das als Kinase-Substrat verwendet wurde, wurde bei der Fa. Sigma gekauft.
CDK2/CycE (50 ng/Messpunkt) wurde für 10 min bei 22°C in Anwesenheit verschiedener Konzentrationen an Testsubstanzen (0 μM, sowie innerhalb des Bereiches 0,001 - 10 μM) in Assaypuffer [50 mM Tris/HCl pH8,0; 10 mM $MgCl_2$; 0,1 mM Na ortho-Vanadat; 1,0 mM Dithiothreitol; 0,5 μM Adenosintrisphosphat (ATP); 10 μg/Messpunkt Histon IIIS; 0,2 μCi/Messpunkt $^{33}$P-gamma ATP; 0,05% NP40; 1,25% Dimethylsulfoxid] inkubiert. Die Reaktion wurde durch Zugabe von EDTA-Lösung (250 mM; pH8,0; 15 μl/Messpunkt) gestoppt.
Von jedem Reaktionsansatz wurden 15 μl auf P30 Filterstreifen (Fa. Wallac) aufgetragen, und nicht-eingebautes $^{33}$P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLex™ A, Fa. Wallac) bedeckt und für 1 Stunde bei 90°C eingebrannt. Die Menge an eingebautem $^{33}$P (Substratphosphorylierung) wurde durch Szintillationsmessung in einem Gamma-Strahlungsmessgerät (Wallac) bestimmt. Die Messdaten wurden normalisiert auf 0% Inhibition (Enzymreaktion ohne Inhibitor) und 100% Inhibition (alle Assaykomponenten außer Enzym). Die Bestimmung der IC50 Werte erfolgte mittels eines 4-Parameter Fits unter Benutzung firmeneigener Software.

**Assay 3: CDK4/CycD Kinase Assay**

Rekombinante CDK4- und CycD1-GST-Fusionsproteine, gereinigt aus Bakulovirus-infizierten Insektenzellen (Sf9), wurden von ProQinase GmbH, Freiburg, gekauft. CDK4/CycD1 (250 ng/Messpunkt) wurde für 3 Stunden bei 22°C in Anwesenheit verschiedener Konzentrationen an Testsubstanzen (0 μM, sowie innerhalb des Bereiches 0,001 - 10 μM) in 31 μl Assaypuffer [50 mM Hepes pH7,0; 2,5 mM MnCl; 0,05 mM Na ortho-Vanadat; 1,0 mM Dithiothreitol; 0,25 μM Adenosintrisphosphat (ATP); 0,5 μM biotinyliertes myelin basic protein (bio-MBP, GE Healthcare); 0,05 μCi/Messpunkt $^{33}$P-gamma ATP; 0,005% NP40; 0,025% bovines Serumalbumin; 3% Dimethylsulfoxid] inkubiert. Die Reaktion wurde durch Zugabe von 50 μl Stopp-Mix [100 μM ATP; 10 mM EDTA pH8,0; 0.2% Triton X100; 0.125 mg Streptavidin-SPA Beads (GE Healthcare)] abgestoppt. Nach 10 min Inkubation bei Raumtemperatur wurden die SPA Beads durch Zentrifugation (10 min; 1500g) pelletiert. Die Menge an eingebautem $^{33}$P (Substratphosphorylierung) wurde durch Szintillationsmessung in einem beta-Strahlungsmessgerät (Microbeta, Perkin Elmer) bestimmt. Die Messdaten wurden normalisiert auf 0% Inhibition (Enzymreaktion ohne Inhibitor) und 100% Inhibition (alle Assaykomponenten außer Enzym). Die Bestimmung der IC50 Werte erfolgte mittels eines 4-Parameter Fits unter Benutzung firmeneigener Software.

**Assay 4: VEGF Rezeptor-2 Kinase Assay**

Rekombinante VEGF Rezeptortyrosinkinase-2 wurde als GST-Fusionsprotein aus Bakulovirus-infizierten Insektenzellen (Sf9) gereinigt. Poly-(Glu4Tyr), das als Kinase-Substrat verwendet wurde, wurde bei der Fa. Sigma gekauft. VEGF Rezeptortyrosinkinase (90 ng/Messpunkt) wurde für 10 min bei 22°C in Anwesenheit verschiedener Konzentra-

tionen an Testsubstanzen (0 μM, sowie innerhalb des Bereiches 0,001 - 10 μM) in 30 μl Assaypuffer [40 mM Tris/HCl pH5,5; 10 mM $MgCl_2$; 1 mM $MnCl_2$; 3 μM Na ortho-Vanadat; 1,0 mM Dithiothreitol; 8 μM Adenosintrisphosphat (ATP); 0,96 μg/Messpunkt poly-($Glu_4$Tyr); 0,2 μCi/Messpunkt $^{33}$P-gamma ATP; 1.4% Dimethylsulfoxid] inkubiert. Die Reaktion wurde durch Zugabe von EDTA-Lösung (250 mM; pH8,0; 15 μl/Messpunkt) gestoppt.
Von jedem Reaktionsansatz wurden 15 μl auf P30 Filterstreifen (Fa. Wallac) aufgetragen, und nicht-eingebautes $^{33}$P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLex™ A, Fa. Wallac) bedeckt und für 1 Stunde bei 90°C eingebrannt. Die Menge an eingebautem $^{33}$P (Substratphosphorylierung) wurde durch Szintillationsmessung in einem gamma-Strahlungsmessgerät (Wallac) bestimmt. Die Messdaten wurden normalisiert auf 0% Inhibition (Enzymreaktion ohne Inhibitor) und 100% Inhibition (alle Assaykomponenten außer Enzym). Die Bestimmung der IC50 Werte erfolgte mittels eines 4-Parameter Fits unter Benutzung firmeneigener Software.

**Assay 5: Proliferationsassays**

Kultivierte humane Tumorzellen (MCF7, hormonunabhängige menschliche Mammakarzinomazellen, bezogen von ATCC HTB22; NCI-H460, menschliche nicht kleinzellige Lungenkarzinomazellen, ATCC HTB-177; DU 145, hormonunabhängige menschliche Prostatakarzinomzellen, ATCC HTB-81; HeLa-MaTu, menschliche Cervix-Karzinomzellen, EPO-GmbH, Berlin; HeLa-MaTu-MDR, multiple Arzneimittel resistente menschliche Cervix-Karzinomzellen, EPO-GmbH, Berlin; Caco-2, menschliche Colon-Karzinomazellen, ATCC HTB-37; B16F10, murine Melanomzelle, ATCC CRL-6475) wurden in einer Dichte von ca. 1000-5000 Zellen/Messpunkt, je nach Wachstumsgeschwindigkeit der jeweiligen Zellen, in einer 96-Loch Multititerplatte in 200 μl des entsprechenden Wachstumsmediums ausplattiert. Nach 24 Stunden wurden die Zellen einer Platte (Nullpunkt-Platte) mit Kristallviolett gefärbt (s.u.), während das Medium der anderen Platten durch frisches Kulturmedium (200 μl), dem die Testsubstanzen in verschiedenen Konzentrationen (0 μM, sowie im Bereich 0,003 - 3 μM; die finale Konzentration des Lösungsmittels Dimethylsulfoxid betrug 0,5%) zugesetzt waren, ersetzt. Die Zellen wurden für 4 Tage in Anwesenheit der Testsubstanzen inkubiert. Die Zellproliferation wurde durch Färbung der Zellen mit Kristallviolett bestimmt: Die Zellen wurden durch Zugabe von 20 μl/Messpunkt einer 11 %igen Glutaraldehyd-Lösung 15 min bei Raumtemperatur fixiert. Nach dreimaligem Waschen der fixierten Zellen mit Wasser wurden die Platten bei Raumtemperatur getrocknet. Die Zellen wurden durch Zugabe von 100 μl/Messpunkt einer 0,1%igen Kristallviolett-Lösung (pH durch Zugabe von Essigsäure auf pH3 eingestellt) gefärbt. Nach dreimaligem Waschen der gefärbten Zellen mit Wasser wurden die Platten bei Raumtemperatur getrocknet. Der Farbstoff wurde durch Zugabe von 100 μl/Messpunkt einer 10%igen Essigsäure-Lösung gelöst. Die Extinktion wurde photometrisch bei einer Wellenlänge von 595 nm bestimmt. Die Messdaten wurden normalisiert auf 0% Inhibition [unbehandelte (0 μM) Zellen] und 100% Inhibition (Extinktionwerte der Nullpunktplatte). Die Bestimmung der IC50 Werte erfolgte mittels eines 4-Parameter Fits unter Benutzung firmeneigener Software.

**Assay 6: Permeabilitätsassays**

Der Caco-2 Monolayer stellt eine Barriere zwischen 2 Kompartimenten dar. Die Zellen verhalten sich dabei ähnlich der Dünndarmzellen. Wirkstoffe können sowohl para- wie auch transzellulär transportiert werden. Dabei erfolgt der Transport meist von *apikal* (*luminal*) nach *basolateral* (*serosal*). Bei p-Glycoproteinsubstraten wird in der Regel auch der Rücktransport von *basolateral* nach *apikal* beobachtet.
Versuchsdurchführung: Der Permeabilitätsversuch wird sowohl von *apikal* nach *basolateral* als auch von *basolateral* nach *apikal* durchgeführt. Dabei werden pro Substanz 2 Filter verwendet; die Inkubation erfolgt über 90 min bei 37°C im Wasserbad. Neben der bidirektionalen Permeabilität von Test- und Referenzsubstanzen (Referenzen: geringe Permeabilität: PEG4000; hohe Permeabilität: Clonidin; direktionale Permeabilität: Digoxin), wird durch die Bestimmung des transepithelialen Widerstandes (TEER) die Integrität des Zellmonolayers sichergestellt. Als Referenzsubstanzen werden (i) PEG 4000 als hydrophiler Marker benutzt. Es ist wegen seines hohen Molekulargewichtes unfähig in die Zellmembran oder in die Poren der Tight junctions zu permeieren. PEG 4000 ist daher ein Marker für die Unversehrtheit des Zellmonolayers und die Enge der Tight junctions. PEG 4000 wird im Menschen nicht absorbiert. (ii) Clonidine ist bekannt als vollständig absorbierte Substanzen beim Menschen (100 %). Sie dienen als Marker für sehr gut-permeable Substanzen mit Papp-Werten über 100 nm/s. (iii) Digoxin ist ein bekanntes Pgp Substrat. Es zeigt eine geringe Permeabilität von *apikal* nach *basolateral.* Beim Rückversuch (*basolateral* nach *apikal*) sollten die Papp-Werte bedingt durch den aktiven direktionalen Transport ins *apikale* Kompartment um ca. Faktor 10 höher liegen.
Auswertung: Der Permeationskoeffizient (Papp) wird über die Substanzkonzentration auf der Donor- und Rezeptorseite nach folgender Formel berechnet:

$$Papp = (Vc\ res / A * C_{t0,\ don}) * (delta\ C_{res}/delta\ T),$$

wobei

| | |
|---|---|
| Vres: | Puffervolumen auf der Rezeptorseite, |
| A: | Filterfläche = 1 $cm^2$, |
| $C_{to,\ don}$: | Substanzkonzentration auf der Donorseite zum Zeitpunkt 0, |
| delta $C_{res}$/delta T: | Änderung der Substanzkonzentration über die Zeit auf der Rezeptorseite. |

Der Permeationskoeffizient Papp wird zur Einschätzung der Absorption beim Menschen nach folgendem Schema genutzt:

| Permeationskoeffizient Papp [nm/s] | geschätzte Absorption |
|---|---|
| >1 / <10 | Schlechte Absorption, bevorzugt parazellulär über die tight junctions (Bsp.: Mannitol, Sucrose, Cimetidine) |
| >10 / <60 | Mittelmäßige Absorption, bevorzugt transzellulär |
| >60 / >100 | Gute Absorption, bevorzugt transzellulär (Bsp.: Clonidine, Testosteron) |

**Ergebnisse aus den Enzym- und Zellassays**

Tab.1: Ergebnisse der Enzymassays

| Bsp. | CDK1/CycB (Assay 1) | CDK2/CycE (Assay 2) | CDK4/CycD (Assay 3) | VEGF-R2 (Assay 4) |
|---|---|---|---|---|
| | Konzentration der halbmaximalen Inhibition der Enzymaktivität, IC50 [nM] | | | |
| 1 | 16 | 16 | 20 | 260 |
| 2 | 9 | 8 | 24 | 480 |
| 3 | 32 | 12 | 78 | 490 |
| | | | | |
| Struktur 692 des Beispiels 1 aus WO 2003/032997 | 53 | 55 | >1000 | 120 |

Tab.2: Ergebnisse der Proliferationsassays (Assay 5)

| Bsp. | HeLa-MaTu | HeLa-MaTu-ADR | MCF7 | NCI-H460 | DU145 | Caco-2 | B16F10 |
|---|---|---|---|---|---|---|---|
| | Konzentration der halbmaximalen Inhibition der Zellproliferation, IC50 [nM] | | | | | | |
| 1 | 11 | 9 | 23 | 41 | 17 | 45 | 49 |
| 2 | 14 | 14 | 35 | 38 | 34 | 66 | 70 |
| 3 | 35 | 39 | 160 | 140 | 74 | 180 | 270 |
| | | | | | | | |
| Struktur 692 des Beispiels 1 aus WO 2003/032997 | 150 | 1200 | 71 | 290 | 510 | 1400 | 450 |

Tab.3: Ergebnisse der Permeabilitätsassays (Assay 6)

| Bsp. | apikal-basolateral (a-b) | basolateral-apikal (b-a) | ratio (b-a)/(a-b) |
|---|---|---|---|
| | Permeationskoeffizient, Papp [nM/s] | | |
| 1 | 165 | 147 | 0.9 |
| 2 | 196 | 194 | 1.0 |
| | | | |
| Struktur 692 des Beispiels 1 aus WO 2003/032997 | 9 | 294 | 32 |

**Schlußfolgerungen aus den Enzym- und Zellassays**

Die Beispielverbindungen 1-3 zeigen eine 2- bis 6-fach stärke Inhibition der Aktivität der Zyklin-abhängigen Kinase CDK1 und eine 3- bis 7-fach stärkere Inhibition der CDK2 im Vergleich zu der Struktur 692 des Beispiels 1 aus WO 2003/032997 (Tab.1). Die Beispielverbindungen 1-3 zeigen eine potente Inhibition der CDK4 bei nanomolaren Konzentrationen während die Struktur 692 des Beispiels 1 aus WO 2003/032997 die halb-maximale Hemmung der CDK4 Aktivität bei zu einer Konzentration von 1000 nM noch nicht erreicht hat. Gleichzeitig ist die Selektivität hinsichtlich der CDK-Inhibition gegenüber der Inhibition der VEGF-Rezeptorkinase-2 (VEGF-R2) bei den Beispielen 1-3 deutlich erhöht (15- bis 50-fach stärkere inhibition der CDKs), während die Struktur 692 des Beispiels 1 aus WO 2003/032997 nur eine ca. 2-fache Selektivität aufweist. In den Zellproliferationsassays zeigen die Beispielverbindungen 1-3 die 50%ige Inhibition der Proliferation bereits bei deutlich niedrigeren Konzentrationen als die Struktur 692 des Beispiels 1 aus WO 2003/032997 (Tab. 2, Ausnahme: Bsp. 2 an MCF7 Zellen). Überraschenderweise ist dieser Effekt bei den Zellinien DU145, Caco-2, HeLa-MaTu-ADR, und B16F10 besonders ausgeprägt (bis zu 130-fach bessere antiproliferative Aktivität der Beispielverbindungen 1-3 gegenüber der Struktur 692 des Beispiels 1 aus WO 2003/032997). Die Permeabilitätsassays (Tab. 3) zeigen, dass die Beispielverbindungen 1-2 eine gute und freie Permeation über eine geschlossene Caco-2 Zellschicht aufweisen. Die Struktur 692 des Beispiels 1 aus WO 2003/032997 ist durch eine sehr schlechte Permeation in absorptiver Richtung und durch eine hohe Permeation in Efflux-Richtung gekennzeichnet.

Diese Daten belegen die Überlegenheit der erfindungsgemäßen Verbindungen (Beispiele 1-3) gegenüber dem nächstliegenden Stand der Technik (WO 2003/032997). Dies wird insbesondere deutlich gezeigt anhand der verstärkten antiproliferativen Aktivität der Beispielverbindungen in den als Chemotherapieresistent bekannten Zellinien DU145, HeLa-MaTu-ADR und Caco-2.

**Patentansprüche**

**1.** Verbindungen der allgemeinen Formel (I),

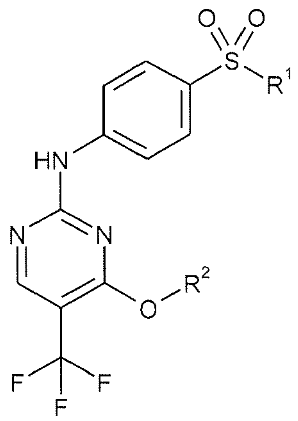

(I),

in der

$R^1$ für einen $C_1$-$C_6$-Alkyl-, $C_2$-$C_6$-Alkenyl-, $C_2$-$C_6$-Alkinylrest oder einen $C_3$-$C_7$-Cycloalkyl oder Phenylring steht, jeweils gegebenenfalls mit Hydroxy, $-NR^3R^4$, Cyano, Halogen, $-CF_3$, $C_1$-$C_6$-Alkoxy, $-OCF_3$ und/oder $C_1$-$C_6$-Alkyl ein- oder mehrfach, gleich oder verschieden substituiert, und

$R^2$ für einen $C_1$-$C_{10}$-Alkyl-, $C_3$-$C_{10}$-Alkenyl- oder $C_3$-$C_{10}$-Alkinylrest oder einen $C_3$-$C_7$-Cycloalkylring steht, jeweils gegebenenfalls ein- oder mehrfach, gleich oder verschieden substituiert mit a) Halogen, Hydroxy, $-NR^3R^4$, Cyano, $-CF_3$, $-OCF_3$, und/oder b) $C_1$-$C_6$-Alkoxy, $C_1$-$C_6$-Alkyl, $C_2$-$C_6$-Alkenyl, $C_2$-$C_6$-Alkinyl, oder $C_3$-$C_8$-Cycloalkyl, $-O-CH_2$-Phenyl, $C_n$-Alkoxycarbonyl, jeweils gegebenenfalls selbst ein- oder mehrfach, gleich oder verschieden mit Halogen, Hydroxy, einem $C_1$-$C_6$-Alkyl, $C_1$-$C_6$- Alkoxy, $-NR^3R^4$, $-CF_3$ und/oder $-OCF_3$ substituiert, und

$R^3$ und $R^4$ unabhängig voneinander für Wasserstoff und/oder einen $C_1$-$C_6$- Alkylrest, $C_2$-$C_6$-Alkenylrest, $C_3$-$C_8$-Cycloalkyl- und/oder Phenylring, einen Heterocyclylring mit 3 bis 8 Ringatomen und/oder einen monocyclischen Heteroarylring, gegebenenfalls mit Hydroxy,- $NR^5R^6$, Cyano, Halogen, $-CF_3$, $C_1$-$C_6$-Alkoxy und/oder $-OCF_3$ ein- oder mehrfach, gleich oder verschieden substituiert sind, oder

$R^3$ und $R^4$ gemeinsam mit dem Stickstoffatom einen 5- bis 7-gliedrigen Ring bilden, der gegebenenfalls zusätzlich zum Stickstoffatom 1 oder 2 weitere Heteroatome enthält und der mit Hydroxy, $-NR^5R^6$, Cyano, Halogen, $-CF_3$, $C_1$-$C_6$-Alkoxy und/oder $-OCF_3$ ein- oder mehrfach, gleich oder verschieden substituiert sein kann, und

$R^5$ und $R^6$ unabhängig voneinander für Wasserstoff oder einen $C_1$-$C_6$-Alkylrest stehen, der gegebenenfalls mit Hydroxy, Cyano, Halogen, $-CF_3$, $C_1$-$C_6$-Alkoxy und/oder $-OCF_3$ ein- oder mehrfach, gleich oder verschieden substituiert ist.

sowie deren Salze, Diatereomere und Enantiomere.

**2.** Verbindungen gemäß Anspruch 1,
wobei

$R^1$ für einen $C_1$-$C_6$-Alkyl-, $C_2$-$C_6$-Alkenyl-, $C_2$-$C_6$-Alkinylrest oder einen $C_3$-$C_7$-Cycloalkyl oder Phenylring steht, jeweils gegebenenfalls mit Hydroxy, Cyano, Halogen, $-CF_3$, $C_1$-$C_6$-Alkoxy, $-OCF_3$ und/oder $C_1$-$C_6$-Alkyl ein- oder mehrfach, gleich oder verschieden substituiert,

sowie deren Salze, Diatereomere und Enantiomere.

**3.** Verbindungen gemäß einem der Ansprüche 1 oder 2,
wobei

$R^2$ für einen $C_1$-$C_{10}$-Alkyl-, $C_3$-$C_{10}$-Alkenyl- oder $C_3$-$C_{10}$-Alkinylrest oder einen $C_3$-$C_7$-Cycloalkylring steht, jeweils gegebenenfalls ein- oder mehrfach, gleich oder verschieden substituiert mit Halogen, Hydroxy, Cyano, $-CF_3$, $-OCF_3$, und/oder $C_1$-$C_6$-Alkoxy, $C_1$-$C_6$-Alkyl, jeweils gegebenenfalls selbst ein- oder mehrfach, gleich oder verschieden mit Halogen oder Hydroxy substituiert,

sowie deren Salze, Diatereomere und Enantiomere.

**4.** Verbindungen gemäß einem der Ansprüche 1 bis 3,
wobei

$R^1$ für einen $C_1$-$C_6$-Alkyl- oder $C_2$-$C_6$-Alkenylrest oder einen $C_3$-$C_7$- Cycloalkyl- oder Phenylring steht, jeweils gegebenenfalls mit Hydroxy, Cyano, Halogen und/oder $C_1$-$C_6$-Alkyl ein- oder mehrfach, gleich oder verschieden substituiert,

sowie deren Salze, Diatereomere und Enantiomere.

**5.** Verbindungen gemäß einem der Ansprüche 1 bis 4,
wobei

$R^2$ für einen $C_2$-$C_6$-Alkyl-, $C_3$-$C_6$-Alkenyl- oder $C_3$-$C_6$-Alkinylrest oder einen $C_3$-$C_7$-Cycloalkylring steht, der gegebenenfalls ein- oder mehrfach substituiert ist mit Hydroxy, Halogen, $-CF_3$ und/oder $C_1$-$C_3$- Alkoxy,

sowie deren Salze, Diatereomere und Enantiomere.

**6.** Verbindungen gemäß einem der Ansprüche 1 bis 5, wobei

$R^2$ für die Gruppe mit der Teilformel ($I$-$_{R}{}^{2}$) steht,

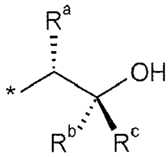

($I$-$_{R}{}^{2}$)

in der

$R^a$ für eine Methyl-, Ethyl-, Propyl- oder Isopropylgruppe steht, und
$R^b$ und $R^c$ unabhängig voneinander für Wasserstoff, eine Methyl- oder Ethylgruppe stehen.

sowie deren Salze, Diatereomere und Enantiomere.

**7.** Verbindungen gemäß Anspruch 6, wobei $R^a$ und $R^b$ für eine Methylgruppe und $R^c$ für Wasserstoff oder eine Methylgruppe stehen, sowie deren Salze, Diatereomere und Enantiomere.

**8.** Verbindungen der allgemeinen Formel (Ia)

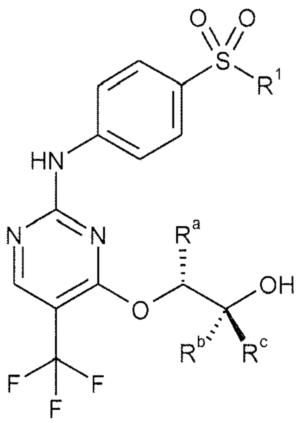

(Ia),

in der

$R^1$ für eine Methylgruppe oder für einen Cyclopropylring steht, und
$R^a$ und $R^b$ für eine Methylgruppe stehen, und
$R^c$ für Wasserstoff oder eine Methylgruppe steht,

sowie deren Salze, Diatereomere und Enantiomere.

**9.** Verfahren zur Herstellung der Verbindungen gemäß einem der Ansprüche 1 bis 8 umfassend mindestens einer der Schritte

$a_1$) Funktionalisierung der 4-Position von 2,4-Dichlor-5-jod-pyrimidin (1) durch Umsetzung mit einem Alkohol der Formel (2) unter Bildung eines Intermediates der Formel (3),

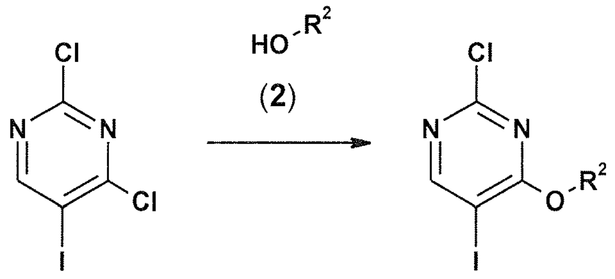


(1) (3)

und nachfolgende Umsetzung von Intermediat (3) unter Bildung des 5-$CF_3$ Intermediates (4)

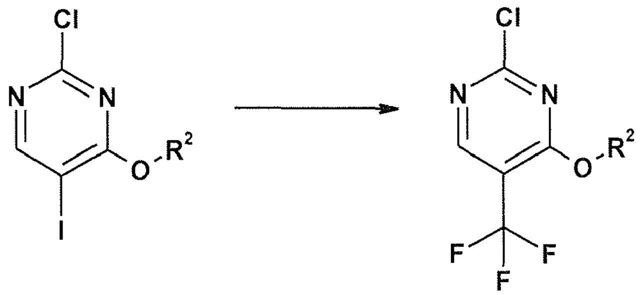

(3) (4)

oder alternativ

$a_2$) direkte Umsetzung von 2,4-Dichlor-5-trifluormethyl-pyrimidin (5) und einem Alkohol der Formel (2) unter Bildung des 5-$CF_3$ Intermediates (4).

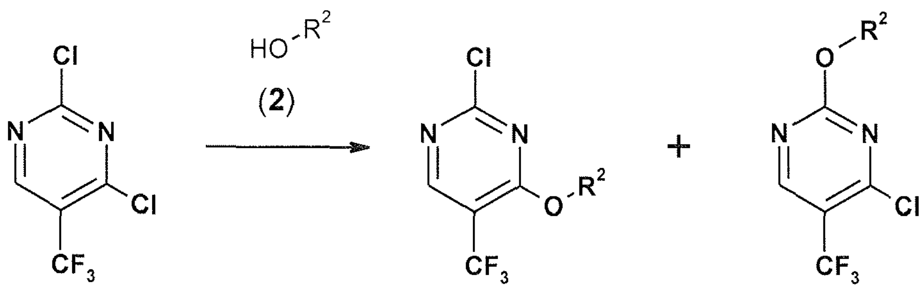


(5) (4) (6)

b) Oxidation eines Thioethers der Formel (7) zum Sulfon der Formel (8).

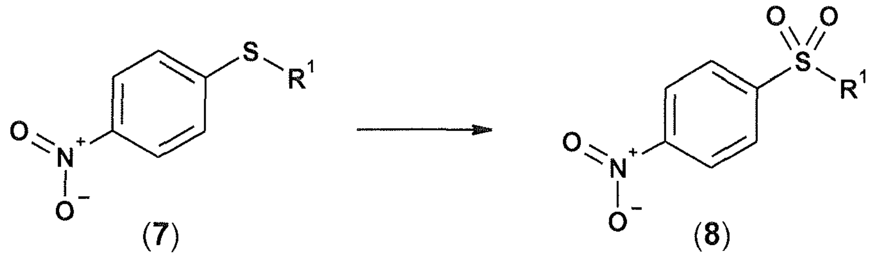

(7) (8)

c) Reduktion der Verbindung der Formel **(8)** zu einer Verbindung der Formel **(9),**

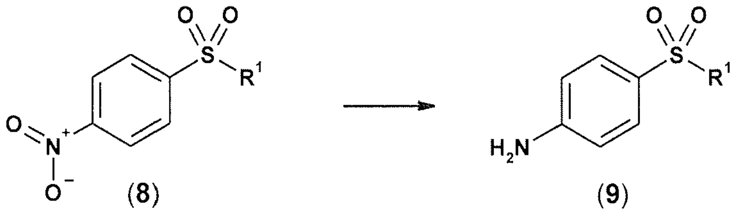

(8) (9)

d) Kupplung der Verbindungen der Formel **(4)** und **(9)**

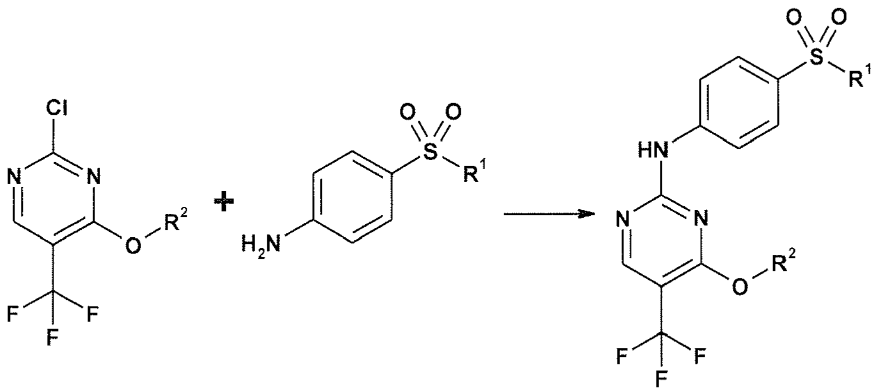

**(4)** **(9)** **(I)**

wobei die Substituenten $R^1$ und $R^2$ die in der allgemeinen Formel (I) gemäß einem der Ansprüche 1 bis 7 angegebenen Bedeutungen aufweisen.

**10.** Verbindungen gemäß einem der Ansprüche 1 bis 8 zur Verwendung als Arzneimittel.

**11.** Verwendung einer Verbindung gemäß einem der Ansprüche 1 bis 8 zur Herstellung eines Arzneimittels zur Behandlung von Krebs.

**12.** Verbindungen gemäß einem der Ansprüche 1 bis 8 zur Verwendung als Arzneimittel gegen Krebs.

**13.** Pharmazeutische Formulierung enthaltend eine Verbindung gemäß einem der Ansprüche 1 bis 8.

Europäisches Patentamt
European Patent Office
Office européen des brevets

# EUROPÄISCHER RECHERCHENBERICHT

Nummer der Anmeldung
EP 08 16 7115

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| D,Y | WO 03/032997 A (BOEHRINGER INGELHEIM PHARMA [DE]; BOEHRINGER INGELHEIM PHARMA [US]; BO) 24. April 2003 (2003-04-24)<br>* Seite 151; Beispiel 692 *<br>----- | 1-13 | INV.<br>C07D239/47<br>A61K31/505<br>A61P35/00 |
| D,Y | WO 2005/037800 A (SCHERING AG [DE]; LUECKING ULRICH [DE]; KRUEGER MARTIN [DE]; JAUTELAT) 28. April 2005 (2005-04-28)<br>* Ansprüche *<br>* Beispiele 1.2,1.4 *<br>----- | 1-13 | |
| D,Y | WO 02/096887 A (ASTRAZENECA AB [SE]; ASTRAZENECA UK LTD [GB]; NEWCOMBE NICHOLAS JOHN [) 5. Dezember 2002 (2002-12-05)<br>* Beispiele 2,7,9,31,40 *<br>----- | 1-13 | |
| A | WO 2008/107096 A (BAYER CROPSCIENCE AG [DE]; GREUL JOERG NICO [DE]; GAERTZEN OLIVER [DE])<br>12. September 2008 (2008-09-12)<br>* Ansprüche *<br>* Beispiel 200 *<br>----- | 1 | RECHERCHIERTE SACHGEBIETE (IPC)<br>C07D<br>A61K<br>A61P |

1

Der vorliegende Recherchenbericht wurde für alle Patentansprüche erstellt

| Recherchenort | Abschlußdatum der Recherche | Prüfer |
|---|---|---|
| München | 3. Februar 2009 | Kollmannsberger, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 08 16 7115

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-02-2009

| Im Recherchenbericht angeführtes Patentdokument | Datum der Veröffentlichung | Mitglied(er) der Patentfamilie | Datum der Veröffentlichung |
|---|---|---|---|
| WO 03032997 A | 24-04-2003 | AT 407678 T | 15-09-2008 |
| | | CA 2463989 A1 | 24-04-2003 |
| | | DK 1438053 T3 | 08-12-2008 |
| | | EP 1438053 A1 | 21-07-2004 |
| | | JP 2005509624 T | 14-04-2005 |
| | | UY 27487 A1 | 30-05-2003 |
| WO 2005037800 A | 28-04-2005 | AU 2004281960 A1 | 28-04-2005 |
| | | BR PI0415421 A | 05-12-2006 |
| | | CA 2542492 A1 | 28-04-2005 |
| | | CN 1867553 A | 22-11-2006 |
| | | DE 10349423 A1 | 16-06-2005 |
| | | EP 1673352 A1 | 28-06-2006 |
| | | JP 2007508354 T | 05-04-2007 |
| | | KR 20060122860 A | 30-11-2006 |
| | | MX PA06004091 A | 27-06-2006 |
| WO 02096887 A | 05-12-2002 | AT 349432 T | 15-01-2007 |
| | | BR 0210071 A | 22-06-2004 |
| | | CA 2448305 A1 | 05-12-2002 |
| | | CN 1541208 A | 27-10-2004 |
| | | DE 60217118 T2 | 28-06-2007 |
| | | DK 1406876 T3 | 02-04-2007 |
| | | EP 1406876 A1 | 14-04-2004 |
| | | ES 2278023 T3 | 01-08-2007 |
| | | JP 2004533458 T | 04-11-2004 |
| | | MX PA03010877 A | 17-02-2004 |
| | | NO 20035210 A | 27-11-2003 |
| | | NZ 529763 A | 27-01-2006 |
| | | US 2004198757 A1 | 07-10-2004 |
| | | ZA 200309165 A | 10-03-2005 |
| WO 2008107096 A | 12-09-2008 | CL 5622008 A1 | 13-06-2008 |
| | | DE 102007010801 A1 | 04-09-2008 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 4029650
- WO 9919305 A
- WO 0114375 A
- WO 9902162 A
- WO 0204429 A
- WO 0012486 A
- WO 0039101 A
- WO 02096888 A
- WO 03076437 A
- WO 2005037800 A
- WO 2003032997 A
- WO 2007071455 A, U. Lücking
- WO 2006081388 A, T.M. Caldwell
- WO 2004048343 A, J. Bryant


### In der Beschreibung aufgeführte Nicht-Patentliteratur


- Remington's Pharmaceutical Science. Mack Publishing Company, 1980
- **P.J. Kocienski.** Protecting Groups. Georg Thieme Verlag Stuttgart, 1994
- **G.E. Carr ; R.D. Chambers ; T.F. Holmes.** *J. Chem. Soc. Perkin Trans.,* 1988, vol. 1, 921
- **F. Cottet ; M. Schlosser.** *Eur. J. Org. Chem.,* 2002, 327
- **F.G. Njoroge et al.** *J. Med. Chem,* 1997, vol. 40, 4290
- **R.C. Larock.** Comprehensive Organic Transformations. VCH, 1989, 411
- **Block et al.** *J. Am. Chem. Soc,* 1992, vol. 114, 3492